(19) Europäisches Patentamt
European Patent Office
Office européen des brevets

(11) EP 4 542 228 A1

(12) **EUROPEAN PATENT APPLICATION**
published in accordance with Art. 153(4) EPC

(43) Date of publication:
**23.04.2025 Bulletin 2025/17**

(21) Application number: **23823782.0**

(22) Date of filing: **06.06.2023**

(51) International Patent Classification (IPC):
***G01N 33/543*** $^{(2006.01)}$ ***C09K 11/06*** $^{(2006.01)}$
***C09K 11/08*** $^{(2006.01)}$ ***G01N 33/545*** $^{(2006.01)}$
***G01N 33/553*** $^{(2006.01)}$

(52) Cooperative Patent Classification (CPC):
**C09K 11/06; C09K 11/08; G01N 33/543; G01N 33/545; G01N 33/553**

(86) International application number:
**PCT/JP2023/021018**

(87) International publication number:
**WO 2023/243490 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.06.2022 JP 2022095905**

(71) Applicants:
- **Tokushima University**
  **Tokushima-shi, Tokushima 770-8501 (JP)**
- **PHC Corporation**
  **Toon-shi**
  **Ehime 791-0395 (JP)**

(72) Inventors:
- **YANO, Takaaki**
  **Tokushima-shi, Tokushima 770-8501 (JP)**
- **KATO, Ryo**
  **Tokushima-shi, Tokushima 770-8501 (JP)**
- **UESUGI, Mitsuhiro**
  **Tokushima-shi, Tokushima 770-8501 (JP)**
- **KOMATSU, Yoshie**
  **Tokushima-shi, Tokushima 770-8501 (JP)**
- **KITAWAKI, Fumihisa**
  **Tokushima-shi, Tokushima 770-8501 (JP)**

(74) Representative: **Eisenführ Speiser**
**Patentanwälte Rechtsanwälte PartGmbB**
**Postfach 10 60 78**
**28060 Bremen (DE)**

# (54) NANOPARTICLE BODY, COMPOSITE CONTAINING NANOPARTICLE BODIES, AND METHOD FOR FORMING POLYMER MEMBRANE CONTAINING NANOPARTICLE BODY

(57) A nanoparticle body comprising: a metallic nanoparticle; a polymer film covering a surface of the metallic nanoparticle; a specifically bondable substance that specifically bonds to a test substance in a specimen; and a fluorescent substance labeled on a surface of the polymer film or on the specifically bondable substance, wherein the fluorescent substance is excited by light having a luminous wavelength of plasmon resonance in a composite in which two or more of the nanoparticle body are bonded together with the test substance interposed therebetween.

Fig.1

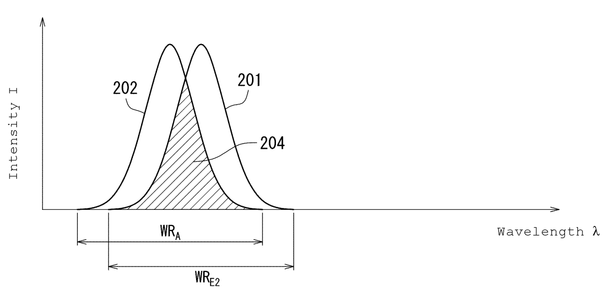


EP 4 542 228 A1

## Description

### TECHNICAL FIELD

The present invention relates to a nanoparticle body (particularly, a nanoparticle body to be used for plasmon-excited fluorescence analysis), a composite containing the nanoparticle body, and a method for forming a polymer film contained in the nanoparticle body.

### BACKGROUND ART

A biosensor makes a specific test substance to be detected specifically react with a specific specifically bondable substance to form a composite, and detects the test substance by a signal caused by a specific bond in the composite.

In plasmon-excited fluorescence analysis, the composite comprises, for example, a test substance, a specifically bondable substance, a fluorescent substance, and a metallic particle. When the composite is irradiated with excitation light, surface plasmon resonance is induced in the metallic particles in the composite, and a near field is formed in the vicinity of the surface of the metallic particle. The near field increases the fluorescence intensity of the fluorescent substance.

The composite particle for immunochromatogram described in Patent Document 1 is composed of a fine particle that has a structure in which the exterior of a fine particle made of metal is covered with at least one layer of silica containing at least one fluorescent substance, and has a surface modified with a labeling substance that specifically recognizes a target substance. In the composite particle of Patent Document 1, the surface of the metallic particle is covered with the silica layer, and the fluorescent substance is immobilized to the silica layer, whereby the fluorescent substance excited by a near field formed by surface plasmon resonance is prevented from coming into contact with the metallic particle. Accordingly, quenching of the excited fluorescent substance is inhibited.

### PRIOR ART DOCUMENT

### PATENT DOCUMENT

Patent Document 1: JP-A-2011-220705

### SUMMARY OF THE INVENTION

### PROBLEMS TO BE SOLVED BY THE INVENTION

Incidentally, the present inventors have found through intensive studies that the sensor as described above has room for further improvement in detection sensitivity. Specifically, conventionally, in the selection of the fluorescent substance, the relationship between the absorption spectrum of the fluorescent substance and the luminous wavelength of plasmon resonance is not considered, or even if the relationship is mentioned, the relationship between the absorption spectrum of the fluorescent substance and the luminous wavelength of plasmon resonance derived from the single particle metallic nanoparticle is considered. Therefore, the fluorescence emitted by the fluorescent substance in the composite was not sufficiently enhanced by plasmon resonance.

The present invention has been devised in light of the above problems. That is, a main object of the present invention is to provide a nanoparticle body in which an appropriate fluorescent substance is selected and fluorescence emitted by the fluorescent substance in the composite is sufficiently enhanced by plasmon resonance, and thereby detection sensitivity is increased.

### SOLUTIONS TO THE PROBLEMS

The nanoparticle according to one embodiment of the present invention is a nanoparticle body comprising:

a metallic nanoparticle;
a polymer film covering a surface of the metallic nanoparticle;
a specifically bondable substance that specifically bonds to a test substance in a specimen; and
a fluorescent substance labeled on a surface of the polymer film or on the specifically bondable substance, wherein the fluorescent substance is excited by light having a luminous wavelength of plasmon resonance in a composite in which two or more of the nanoparticle body are bonded with the test substance interposed therebetween.

The composite according to another embodiment of the present invention comprises

two or more of the nanoparticle body, wherein the two or more nanoparticle bodies include a first nanoparticle body and a second nanoparticle body, and
the first nanoparticle body and the second nanoparticle body are bonded with the test substance interposed therebetween.

The method for forming a polymer film according to another embodiment of the present invention comprises: a step of bringing a polymer having a disulfide linkage in a side chain into contact with a metallic nanoparticle to form a polymer film in which the polymer is bonded to a surface of the metallic nanoparticle with a sulfur atom interposed therebetween.

EFFECTS OF THE INVENTION

The present invention can provide a nanoparticle body in which an appropriate fluorescent substance is selected and fluorescence emitted by the fluorescent substance in the composite is sufficiently enhanced by plasmon resonance, and thereby detection sensitivity is increased.

BRIEF DESCRIPTION OF THE DRAWINGS

[Fig. 1] Fig. 1 is a conceptual diagram showing a plasmon resonance spectrum derived from metallic nanoparticles in a single particle state and an absorption spectrum of a fluorescent substance.
[Fig. 2] Fig. 2 is a conceptual diagram showing a plasmon resonance spectrum derived from metallic nanoparticles in a single particle state, a plasmon resonance spectrum derived from metallic nanoparticles in a composite, and an absorption spectrum of a fluorescent substance.
[Fig. 3] Fig. 3 is a conceptual diagram showing a plasmon resonance spectrum derived from metallic nanoparticles in a single particle state, a plasmon resonance spectrum derived from metallic nanoparticles in a composite, and an absorption spectrum of a fluorescent substance.
[Fig. 4] Fig. 4 is a sectional view schematically illustrating a nanoparticle body according to the first embodiment.
[Fig. 5] Fig. 5 is an enlarged sectional view of the portion A in Fig. 4.
[Fig. 6] Fig. 6 is a schematic view illustrating a method for forming a polymer film 3.
[Fig. 7] Fig. 7 is a conceptual diagram showing a plasmon resonance spectrum derived from metallic nanoparticles in a single particle state, a plasmon resonance spectrum derived from metallic nanoparticles in a composite, and a fluorescence spectrum of a fluorescent substance.
[Fig. 8] Fig. 8 is a conceptual diagram showing a plasmon resonance spectrum derived from metallic nanoparticles in a single particle state, a plasmon resonance spectrum of metallic nanoparticles in a composite, and an absorption spectrum and a fluorescence spectrum of a fluorescent substance.
[Fig. 9] Fig. 9 is a sectional view schematically illustrating a composite.
[Fig. 10] Fig. 10 is a sectional view schematically illustrating a composite.
[Fig. 11] Fig. 11 is a sectional view schematically illustrating a composite composed of two nanoparticle bodies.
[Fig. 12] Fig. 12 is a sectional view schematically illustrating a composite composed of three nanoparticle bodies.
[Fig. 13] Fig. 13 is a diagram illustrating a measuring device.
[Fig. 14] Fig. 14 is a schematic diagram illustrating the method for forming a polymer film of Example 1.
[Fig. 15] Fig. 15 is a schematic diagram illustrating the method for forming a polymer film of Example 1.
[Fig. 16] Fig. 16 is a schematic diagram illustrating a structure of a nanoparticle body to which a fluorescently labeled antibody of Example 1 is bonded.
[Fig. 17] Fig. 17 is a schematic diagram illustrating an immunochromatography strip.
[Fig. 18] Fig. 18 is a diagram showing plasmon resonance spectra of Example 1 ((a) preparation for blank and (b) preparation for measurement).
[Fig. 19] Fig. 19 is a diagram illustrating a plasmon resonance spectrum of Example 1, and an absorption spectrum and a fluorescence spectrum of a Ru complex.
[Fig. 20] Fig. 20 is a diagram showing plasmon resonance spectra of Example 2 ((a) preparation for blank and (b) preparation for measurement).
[Fig. 21] Fig. 21 is a diagram illustrating a plasmon resonance spectrum of Example 2, and an absorption spectrum and a fluorescence spectrum of a Ru complex.
[Fig. 22] Fig. 22 is a diagram illustrating an absorption spectrum and a fluorescence spectrum of a fluorescent

substance of Example 3, and a plasmon resonance spectrum of Example 1.

[Fig. 23] Fig. 23 shows a fluorescence spectrum of a test substance-nanoparticle body system of Example 3.

[Fig. 24] Fig. 24 shows a plasmon resonance spectrum of Comparative Example 1 ((a) silica-covered silver nanoparticles in a primary particle state and (b) an aggregate in which three silica-covered silver nanoparticles are arranged in a substantially linear form).

[Fig. 25] Fig. 25 shows a plasmon resonance spectrum of Comparative Example 2 ((a) silica-covered silver nanoparticles in a primary particle state and (b) an aggregate in which three silica-covered silver nanoparticles are arranged in an ozone molecule shape).

## DETAILED DESCRIPTION

Hereinafter, a nanoparticle body, a composite, and a measuring device which are embodiments of the present invention will be described in detail with reference to the embodiments shown in the drawings. Note that the drawings include some schematic ones and may not reflect actual dimensions or proportions.

The numerical ranges referred to in the present description are intended to include the lower and upper limits themselves unless specific terms such as "less than, fewer than, smaller than" and "exceeding, more than, larger than" are attached. That is, taking a numerical range of 1 nm to 10 nm as an example, the numerical range is interpreted as including the lower limit value "1 nm" and the upper limit value "10 nm".

In the present description, the phrase "the object member is substantially constituted of a specific material or that the object member is made of a specific material" means that the object member contains the specific material in a ratio of 95% by mass or more, 97% by mass or more, 99% by mass or more, or 100% by mass. For example, a "nanoparticle constituted of gold" means that the nanoparticle contains gold in a ratio of 95% by mass or more, 97% by mass or more, 99% by mass or more, or 100% by mass.

### <First Embodiment: Nanoparticle Body>

### [Basic Configuration of Nanoparticle Body]

The nanoparticle body according to the first embodiment comprises a metallic nanoparticle, a polymer film, a specifically bondable substance, and a fluorescent substance. The metallic nanoparticle causes plasmon resonance by irradiation with excitation light. The polymer film covers the surface of the metallic nanoparticle. The specifically bondable substance is specifically bonded to a test substance in a specimen, so that a composite is formed. The fluorescent substance is labeled on the surface of the polymer film or on the specifically bondable substance, and emits fluorescence derived from plasmon resonance.

### (Method for Detecting Test Substance Using Nanoparticle Body)

First, for convenience of description of the nanoparticle body according to the present embodiment, for the purpose of aiding understanding thereof, a method for detecting a test substance using the nanoparticle body according to the present embodiment will be described.

The nanoparticle body according to the present embodiment comprises a metallic nanoparticle, a polymer film covering the surface of the metallic nanoparticle, a specifically bondable substance that is specifically bonded to a test substance in a specimen, and a fluorescent substance labeled on the surface of the polymer film or on the specifically bondable substance. The nanoparticle body according to the present embodiment is dispersed in a specimen, and a test substance contained in the specimen is captured to form a composite (fourth embodiment). More specifically, the composite is formed by specifically bonding of the specifically bondable substance of the nanoparticle body with the test substance. The composite has a structure in which, for example, two nanoparticle bodies are bonded together with the test substance interposed therebetween. In the composite, for example, two metallic nanoparticles are disposed apart from each other at a certain distance as a result of bonding to the same test substance with their respective specifically bondable substances.

In surface plasmon fluorescence spectroscopy (SPFS), irradiation of the composite with excitation light causes localized surface plasmon resonance (LSPR) (hereinafter also referred to simply as "plasmon resonance"), so that a near field is efficiently formed near the surfaces of the metallic nanoparticles (in particular, near the surfaces located between two metallic nanoparticles). The near field and the dipole-dipole mechanisms efficiently excite the fluorescent substance of the composite and enhance fluorescence. By measuring the quantity of fluorescence, the test substance in the specimen can be detected.

[Background to the Present Invention]

Next, for convenience of description of the nanoparticle body according to the present embodiment, for the purpose of aiding understanding thereof, the background to the present invention will be described in detail with reference to Figs. 1 to 3. Fig. 1 is a conceptual diagram showing a plasmon resonance spectrum derived from metallic nanoparticle in a single particle state and an absorption spectrum of a fluorescent substance. As illustrated in Fig. 1, the plasmon resonance spectrum 201 derived from metallic nanoparticles in a single particle state has, for example, one peak as a second luminous wavelength range $WR_{E2}$. The absorption spectrum 202 of the fluorescent substance has, for example, one peak as an absorption wavelength range $WR_A$. When the fluorescent substance in the composite is excited by plasmon resonance, the fluorescent substance is considered to be excited by a dipole-dipole mechanism (Förster mechanism (Foerster Resonance Energy Transfer, Fluorescence Resonance Energy Transfer: FRET)) and a near field. For this reason, as illustrated in Fig. 1, the fluorescent substance was selected such that the overlap 204 of the plasmon resonance spectrum 201 derived from the metallic nanoparticles in a single particle state with the absorption spectrum 202 of the fluorescent substance (the overlap of the absorption wavelength range $WR_A$ with the second luminous wavelength range $WR_{E2}$) was large. Conventionally, the fluorescent substance in the composite has been selected in this manner from the viewpoint of increasing detection sensitivity.

As a result of intensive studies, the inventors of the present application have found that conventional selection of a fluorescent substance has the following problems.

Plasmon resonance derived from metallic nanoparticles in a single particle state (that is, plasmon resonance induced in single-particle metallic nanoparticles) is dipole resonance, and plasmon resonance induced in a composite is plasmon resonance derived from dipole-dipole interaction (higher order resonance, that is, multipole resonance). Examples of the multipole resonance include quadrupole resonance. That is, the plasmon resonance mainly contributing to the detection of a test substance is multipole resonance induced by the proximity between the metallic nanoparticles in the composite. Therefore, to efficiently increase detection sensitivity in the detection of a test substance, it is necessary to enhance not fluorescence caused by dipole resonance but fluorescence mainly caused by multipole resonance.

Fig. 2 is a conceptual diagram showing a plasmon resonance spectrum 201 derived from metallic nanoparticles in a single particle state, a plasmon resonance spectrum 206 derived from metallic nanoparticles in a composite, and an absorption spectrum 202 of a fluorescent substance. As shown in Fig. 2, the plasmon resonance spectrum of the metallic nanoparticles in the composite (hereinafter, also referred to as multipole resonance spectrum) 206 has, for example, one peak as a first luminous wavelength range $WR_{E1}$, and is located on the longer wavelength side as compared with the peak of the plasmon resonance spectrum derived from the metallic nanoparticles in a single particle state (hereinafter, also referred to as dipole resonance spectrum) 201. That is, the first luminous wavelength range $WR_{E1}$ of the plasmon resonance is located on the longer wavelength side than the second luminous wavelength range $WR_{E2}$ of the plasmon resonance derived from the metallic nanoparticles in a single particle state (that is, plasmon resonance induced in single-particle metallic nanoparticles). That is, there is no overlap of the absorption spectrum 202 of the fluorescent substance with the plasmon resonance spectrum 206 derived from the metallic nanoparticles in the composite (overlap of the absorption wavelength range $WR_A$ with the first luminous wavelength range $WR_{E1}$).

From the viewpoint of increasing the detection sensitivity for a test substance, the present inventors can say that the spectrum overlap 204 (described in Fig. 1) is an apparent overlap. From the viewpoint of increasing the detection sensitivity for a test substance, it has been found that it is important to select a fluorescent substance such that a first region $R_1$ is made to exist, and it is preferably important to select a fluorescent substance such that the overlap 208 of the multipole resonance spectrum 206 with the absorption spectrum 202 of the fluorescent substance is made larger (see Fig. 3 described later). Here, as illustrated in Fig. 3 to be described later, the first region $R_1$ refers to a region where the multipole resonance spectrum 206 and the absorption spectrum 202 of the fluorescent substance overlap with each other (a region corresponding to the overlap 208).

Fig. 3 is a conceptual diagram showing a plasmon resonance spectrum 201 derived from metallic nanoparticles in a single particle state, a plasmon resonance spectrum 206 derived from metallic nanoparticles in a composite, and an absorption spectrum 202 of a fluorescent substance. In the present embodiment, the fluorescent substance is selected such that there is an (preferably larger) overlap 208 of the absorption spectrum 202 of the fluorescent substance with the resonance spectrum 206 derived from the metallic nanoparticle in the composite. As described above, when the absorption wavelength range $WR_A$ of the fluorescent substance overlaps with the first luminous wavelength range $WR_{E1}$ of plasmon resonance, the fluorescent substance is sufficiently excited and fluorescence is enhanced, so that detection sensitivity is greatly improved. For these reasons, it is considered that the nanoparticle body according to the present embodiment can enhance detection sensitivity.

Based on such technical findings, the present inventors have examined a specific means for enhancing the detection sensitivity, focusing on adjusting the spectral characteristics of the fluorescent substance. As a result, the present inventors have arrived at a characteristic that "a fluorescent substance is excited by light having a luminous wavelength of localized surface plasmon resonance in a composite in which two or more nanoparticle bodies are bonded

with a test substance interposed therebetween".

The nanoparticle according to a first embodiment is a nanoparticle body comprising:

a metallic nanoparticle;
a polymer film covering a surface of the metallic nanoparticle;
a specifically bondable substance that specifically bonds to a test substance in a specimen; and
a fluorescent substance labeled on a surface of the polymer film or on the specifically bondable substance, wherein the fluorescent substance is excited by light having a luminous wavelength of localized surface plasmon resonance in a composite in which two or more of the nanoparticle body are bonded with the test substance interposed therebetween.

[Mechanism of Action]

The nanoparticle body according to the present embodiment can enhance detection sensitivity. Without being bound by a particular theory, the reason for this is presumed as follows. In the nanoparticle body according to the present embodiment, a fluorescent substance is excited by light having a luminous wavelength of plasmon resonance in a composite in which two or more nanoparticle bodies are bonded with a test substance interposed therebetween. As a result, the absorption spectrum of the fluorescent substance and the plasmon resonance spectrum derived from the metallic nanoparticles in the composite overlap with each other, and fluorescence for detecting a test substance is induced by the Förster mechanism and a near field (hereinafter, such fluorescence is also referred to as "excitation induced type fluorescence"). Therefore, since the fluorescent substance is sufficiently excited by light having a luminous wavelength of plasmon resonance derived from the composite, the detection sensitivity can be enhanced.

In addition, fluorescence for detecting a test substance is also induced by an overlap of a fluorescence spectrum of the fluorescent substance with a plasmon resonance spectrum derived from the metallic nanoparticles in the composite (hereinafter, such fluorescence is also referred to as "luminescence induced type fluorescence"). The luminescence induced type fluorescence will be described in detail in the second embodiment.

[Scheme 1]

The fluorescence enhancement in the present embodiment is further described with reference to Scheme 1 of the process of detecting a test substance using the nanoparticle body according to the present embodiment:

[Chemical Formula 1]

(1) $M \xrightarrow{h\nu\ (\text{External irradiation light})} M^*$ : Plasmon resonance (Multipole resonance)

(2) $M^* + Flu(S_0) \longrightarrow M + Flu^*(S_1) +$ Resonant scattering light emission
: Excitation of fluorescent substance

(3) $Flu^*(S_1) \longrightarrow Flu(S_0) +$ Fluorescence : Relaxation of exited fluorescent substance (Fluorescence emission)

in the elementary processes (1) to (3) of Scheme 1, $S_0$ denotes a ground state, $S_1$ denotes an excited singlet state, * denotes an excited state, Flu denotes a fluorescent substance (in the composite), and M denotes a metallic nanoparticle (in the composite).

Scheme 1 includes elementary processes (1) to (3).

As shown in the elementary process (1), the fluorescent substance is excited by light having a luminous wavelength of plasmon resonance in a composite in which two or more nanoparticle bodies are bonded with a test substance interposed therebetween. When the composite formed by capturing a test substance is irradiated with light externally applied as excitation light (hereinafter, also referred to as "external irradiation light"), plasmon resonance (multipole resonance) is induced (that is, the plasmon resonance in the composite is induced by light externally applied). Examples of the test substance include a test substance derived from a specimen which is blood, plasma, urine, or saliva.

As shown in the elementary process (2), the fluorescent substance is excited by a dipole-dipole mechanism and a near field. When the absorption wavelength range $WR_A$ of the fluorescent substance overlaps with the first luminous wavelength range $WR_{E1}$ of plasmon resonance, the fluorescent substance is efficiently excited.

As shown in the elementary process (3), the fluorescent substance in the excited state relaxes and emits fluorescence.

In a preferred embodiment, the maximum absorption wavelength of the fluorescent substance in the first luminous wavelength range $WR_{E1}$ is located at 500 to 700 nm (more preferably 550 to 700 nm). That is, the maximum absorption wavelength of the fluorescent substance is located at 500 to 700 nm in the first luminous wavelength region $WR_{E1}$ of the plasmon resonance spectrum in the composite. Examples of the fluorescent substance include fluorescein derivatives, rhodamine derivatives, cyanine dyes, and Alexa Flouor (registered trademark) manufactured by Molecular Probes. Among them, examples of the fluorescent substance having a maximum absorption wavelength of 500 to 700 nm include 532, 546, 555, 568, 594, and 640 of Alexa Flour (registered trademark) series. The maximum absorption wavelength can be determined as follows. An absorption spectrum of an aqueous solution of a fluorescent substance (solvent: deionized water) is measured, and a peak position of the absorption spectrum obtained is defined as a maximum absorption wavelength.

In another preferred embodiment, when a composite contains two nanoparticle bodies 1, a fluorescent substance is positioned between a first nanoparticle body and a second nanoparticle body in the composite.

Hereinafter, the configuration of a nanoparticle body will be described. With reference to Fig. 4, the nanoparticle body will be described. Fig. 4 is a sectional view schematically illustrating the nanoparticle body according to the present embodiment. The nanoparticle body 1 according to the present embodiment comprises a metallic nanoparticle 2, a polymer film 3 covering the surface of the metallic nanoparticle 2, a specifically bondable substance 4 that is specifically bonded to a test substance in a specimen, and a fluorescent substance 6 labeled on the surface of the polymer film 3.

The nanoparticle body 1 can be used for plasmon-excited fluorescence analysis. In other words, the nanoparticle body 1 can be used for surface plasmon-field enhanced fluorescence spectroscopic immunoassay. The nanoparticle body 1 can capture a test substance in a specimen and form a composite containing two or more nanoparticle bodies 1 and the test substance. When the composite is irradiated with excitation light, plasmon resonance is caused to form a near field. The near field and a dipole-dipole mechanism enhance fluorescence. For example, the nanoparticle body 1 captures one test substance in a specimen and form a composite containing two nanoparticle bodies 1 and the test substance. In such a case, the nanoparticle body 1 includes a first nanoparticle body and a second nanoparticle body as nanoparticle bodies, and forms a composite in which the first nanoparticle body and the second nanoparticle body are bonded with the test substance interposed therebetween.

The nanoparticle body 1 may be blocked with a blocking agent at a nonspecific binding site. The blocked nanoparticle body 1 is inhibited from forming a nonspecific bond to a substance other than the detection target of the specifically bondable substance 4 (that is, a substance other than the test substance) to reduce the background and the false positive signal, and can improve the signal-noise ratio (SN ratio). Examples of the blocking agent include proteins such as bovine serum albumin (BSA), skim milk, and casein, and chemically synthesized polymers.

When the nanoparticle body 1 is present in a solvent, the dispersion of the nanoparticle body 1 may further contain a dispersant for the purpose of improving the dispersibility of the nanoparticle body 1. Examples of such a dispersant include sodium heparin.

(Metallic Nanoparticle)

The metallic nanoparticle 2 is covered on the surface thereof with a polymer film 3. The metallic nanoparticle 2 interacts with light having a specific wavelength, which varies depending on the type of metal, and causes plasmon resonance. There is a plasmon resonance peak in a range from 400 nm to 530 nm for silver nanoparticles, and from 510 nm to 580 nm for gold nanoparticles. This depends on the particle diameter. For example, nanoparticles made of silver and having a particle diameter of 20 nm resonate with light having a wavelength of 405 nm, and nanoparticles made of gold and having a particle diameter of 20 nm resonate with light having a wavelength of 524 nm. The particle diameter (average primary particle diameter) of the metallic nanoparticles 2 is, for example, 5 nm to 100 nm, 40 nm to 90 nm, or 50 nm to 80 nm. The particle diameter of the metallic nanoparticles 2 can be determined by capturing an image of the metallic nanoparticles 2 using a scanning electron microscope (SEM) or a transmission electron microscope (TEM), measuring the particle diameter of the metallic nanoparticles 2 in the image, and calculating the average value (the number of measurements: for example, at least 10) of a plurality of particle diameters.

The metallic nanoparticle 2 preferably comprises gold or silver, and more preferably comprises silver.

(Polymer Film)

The polymer film 3 covers the surface of the metallic nanoparticle 2. The polymer film 3 functions as a metallic quenching molecular film. In the composite, the polymer film 3 can make a fluorescent substance 6 place apart from the surface of the metallic nanoparticle 2 by at least the thickness of the polymer film 3. Therefore, it is possible to inhibit the excited fluorescent substance 6 from quenching in contact with the surfaces of the metallic nanoparticles 2 (quenching by a Dexter mechanism (Dexter Electron Transfer)), and inhibit a decrease in detection sensitivity. The presence of the polymer film 3 can be confirmed by capturing an image of the nanoparticle body 1 using SEM or TEM, and observing the

nanoparticle body 1 in the image.

The polymer film 3 will be described with reference to Fig. 5. Fig. 5 is an enlarged view of the portion A in Fig. 4, and is an enlarged sectional view of the vicinity of the interface between the polymer film 3 and the surface of the metallic nanoparticle 2 in the nanoparticle body 1. The polymer film 3 contains at least one selected from the group consisting of a binding site 3a with a sulfur atom interposing therein, a positively charged group 3b, and a hydrophobic group 3c, between the polymer film 3 and the surface of the metallic nanoparticle 2. More specifically, the polymer film 3 contains a binding site 3a with a sulfur atom interposing therein, a primary ammonium group ($-NH_3^+$) as the positively charged group 3b, and a hydrophobic group 3c, between the polymer film 3 and the surface of the metallic nanoparticle 2. The binding site 3a binds between the surface of the metallic nanoparticle 2 and the polymer film 3 with a sulfur atom interposed therebetween. The positively charged group 3b forms an electrostatic bond (ionic bond) b with the surface of the negatively charged metallic nanoparticle 2. The hydrophobic group 3c forms a hydrophobic bond c with the surface of the metallic nanoparticle 2.

The polymer film 3 is stably fixed to the surface of the metallic nanoparticle 2 by at least one of the three bonds described above because all of the three bonds bond the surface of the metallic nanoparticle 2 relatively strongly to the polymer film 3. As a result, for example, peeling of the polymer film 3 from the surface of the metallic nanoparticle 2 is prevented. As a result, detachment of the specifically bondable substance 4 associated with the peeling or the like of the polymer film 3 is inhibited, and a decrease in detection sensitivity is inhibited. In addition, exposure of the surface of the metallic nanoparticle 2 due to peeling or the like of the polymer film 3 is inhibited, so that quenching due to contact with an excited fluorescent substance 6 is inhibited and a decrease in detection sensitivity is inhibited. Furthermore, since the polymer film 3 comprises the polymer 3A, the polymer film 3 is easily chemically modified as compared with a silica layer, and the necessity for surface modification or the like is low. As a result, the thickness of the polymer film can be made smaller than that of a silica layer, and the distance between metallic nanoparticles 2 in the composite can be reduced. Therefore, a near field is formed more efficiently, and the detection sensitivity can be improved. For these reasons, the nanoparticle body 1 according to the present embodiment is further superior in detection stability.

As illustrated in Fig. 5, the polymer 3A that constitutes the polymer film 3 can contain at least one selected from the group consisting of a binding site 3a with a sulfur atom interposing therein, a positively charged group 3b, and a hydrophobic group 3c, between the polymer 3A and the surface of the metallic nanoparticles 2. The presence of the binding site 3a with a sulfur atom interposing therein, the positively charged group 3b, and the hydrophobic group 3c can be confirmed by measuring signals derived therefrom using infrared spectroscopy, nuclear magnetic resonance spectroscopy, energy dispersive X-ray spectroscopy (TEM-EDS), X-ray photoelectron spectroscopy (XPS), and time-of-flight secondary ion mass spectrometry (TOF-SIMS). The polymer 3A constituting the polymer film 3 can have a moiety containing a disulfide linkage (-S-S-) as a side chain. The moiety containing a disulfide linkage can have a positively charged group 3b and/or a hydrophobic group 3c.

- Binding Site with Sulfur Atom Interposing Therein -

The binding site 3a with a sulfur atom interposing therein is formed, for example, by mixing a polymer having a moiety containing a disulfide linkage as a side chain with a metallic nanoparticle 2. When the polymer as a raw material has, for example, a hydrophobic group 3c in a side chain with a disulfide linkage interposing therein as shown in Fig. 5, a binding site with a sulfur atom interposing therein is formed between the surface of the metallic nanoparticle 2 and the polymer (see Fig. 5 and the right side in Fig. 15 described later).

- Positively Charged Group -

The polymer film 3 may contain at least a positively charged group 3b in a side chain of the polymer 3A constituting the polymer film 3. The positively charged group 3b forms a relatively strong electrostatic bond with the surface of the metallic nanoparticle 2. In the present description, the positively charged group is a group having a valence of one or more and completely positively ionized. Taking into consideration a plurality of positively charged groups 3b contained in the polymer 3A constituting the polymer film 3, a positively charged group 3b refers to a group having a pKa of 7 or more, the pKa being represented by the following expression (1):

[Math 1]

$$pKa = pH - log\frac{[B]}{[BH^+]} \cdot \cdot \cdot \quad (1)$$

in the expression (1), pKa represents a pKa of a group that is an electrically neutral group contained in the polymer 3A constituting the polymer film 3 and can become a positively charged group (specifically, a primary ammonium group ($-NH_3^+$) or the like) 3b when positively charged (this type of group is also referred to as an electrically neutral group)

(more specifically, a primary amino group ($-NH_2$) or the like); pH represents a pH of an environment where a test substance is detected (more specifically, a specimen or the like); B represents an electrically neutral group contained in the polymer 3A; and $BH^+$ represents a positively charged group 3b contained in the polymer 3A. Namely, the positively charged group 3b refers to a group having a concentration ($[BH^+]$) of the positively charged group being 10 times or more larger than the concentration ([B)) of the electrically neutral group in the case where an equilibrium state represented by the following chemical equilibrium formula (2) is formed in an environment where the electrically neutral group and the positively charged group 3b of the polymer 3A constituting the polymer film 3 detect a test substance (for example, a specimen at a pH of about 6 to 8),

[Chemical Formula 2]

$$B + H^+ \rightleftarrows BH^+ \cdots (2)$$

where in the chemical equilibrium formula (2), $H^+$ denotes a proton, and B and $BH^+$ have the same meanings as B and $BH^+$ in the expression (1).

The positively charged group is, for example, at least one selected from the group consisting of a primary ammonium group, a secondary ammonium group, a tertiary ammonium group, a quaternary ammonium group, and a guanidyl group ($-NHC(=NH_2^+)NH_2$).

- Hydrophobic Group -

The polymer film 3 may contain at least a hydrophobic group 3c in a side chain of the polymer 3A constituting the polymer film 3. The hydrophobic group 3c is, for example, at least one selected from the group consisting of an aromatic cyclic group, an aliphatic cyclic group, and an aliphatic chain group.

Examples of the aromatic cyclic group include an aromatic carbocyclic group and an aromatic heterocyclic group. The aromatic carbocyclic group is a group which does not contain any aromatic heterocyclic ring and contains an aromatic ring in which all ring atoms are carbon atoms. Examples of the aromatic carbocyclic group include aryl groups (more specifically, a phenyl group and the like) and arylalkyl groups (more specifically, a benzyl group and the like). The aromatic heterocyclic group is a group containing an aromatic ring in which at least one of the ring atoms is a hetero atom (more specifically, an oxygen atom, a sulfur atom, a nitrogen atom, or the like). Examples of the aromatic heterocyclic group include nitrogen-containing aromatic heterocyclic groups (more specifically, an imidazoyl group, a pyridyl group (pyridinyl group), and the like), sulfur-containing aromatic heterocyclic groups, and oxygen-containing aromatic heterocyclic groups.

The aliphatic cyclic group is a group which does not contain any aromatic ring and contains a cyclic group composed of a non-aromatic ring. Examples of the aliphatic cyclic group include an aliphatic carbocyclic group and an aliphatic heterocyclic group. The aliphatic carbocyclic group is a group containing a non-aromatic ring in which all ring atoms are carbon atoms, and examples thereof include a cycloalkyl group. An aliphatic heterocyclic group is a group containing a non-aromatic ring in which at least one of the ring atoms is a heteroatom.

The aliphatic chain group is a chain (more specifically, linear or branched) group containing no aromatic ring and no non-aromatic ring. Examples of the aliphatic chain group include aliphatic carbon chain groups (more specifically, an alkyl group, an alkylene group, and the like) and aliphatic heterochain groups.

The polymer 3A constituting the polymer film 3 can form a hydrophobic bond c between the hydrophobic group 3c that the polymer 3A can have and the surface of the metallic nanoparticle 2. The polymer 3A constituting the polymer film 3 can also form other hydrophobic bonds c. For example, a hydrophobic bond c can be formed between a hydrophobic group bonded to the surface of the metallic nanoparticle 2 with a sulfur atom interposing (more specifically, a pyridyl group or the like bonded to the surface of the metallic nanoparticle 2 in Fig. 15 described later with a sulfur atom interposing) 3c and a hydrophobic group which the polymer 3A constituting the polymer film 3 can have (more specifically, an alkylene group possessed by the polymer 3A in Fig. 15) 3c. When such a hydrophobic bond c is formed, the polymer film 3 is more stably fixed to the surface of the metallic nanoparticle 2. The hydrophobic group 3c bonded to the surface of the metallic nanoparticle 2 with a sulfur atom interposing is formed as follows. As described above, the binding site 3a with a sulfur atom interposing therein can be formed, for example, by mixing a polymer having a hydrophobic group 3c in a side chain with a disulfide linkage interposing, with the metallic nanoparticles 2. Here, the hydrophobic group 3c bonded to a sulfur atom is also bonded to the surface of the metallic nanoparticle 2. In this way, a hydrophobic group 3c bonded to the surface of the metallic nanoparticle 2 with a sulfur atom interposing is formed.

The polymer 3A constituting the polymer film 3 may form, with a moiety derived from a crosslinker (a linker moiety) interposing, a linkage with a sulfur atom interposing therein. Examples of such a crosslinker include an amino group-sulfhydryl group crosslinker (more specifically, an NHS-maleimide group crosslinker or the like).

The thickness of the polymer film 3 is preferably 1 nm to 50 nm, and more preferably 1 nm to 10 nm. When the thickness of the polymer film 3 is 50 nm or less, for example, in a composite containing two metallic nanoparticles 2, a separative distance (separation distance) with which a near field is efficiently formed in a space between two metallic nanoparticles 2 is obtained, and thus detection sensitivity is further improved. When the thickness of the polymer film 3 is 1 nm or more, the metallic nanoparticle 2 and the fluorescent substance 6 in the composite are disposed at a prescribed distance, so that quenching of fluorescence emitted from the fluorescent substance 6 excited in measurement is inhibited, and detection sensitivity is further improved.

In the present description, the separative distance (separation distance) refers to the minimum value (shortest distance) of the distance between the surfaces of the metallic nanoparticles 2 contained in two nanoparticle bodies bonded with a test substance interposed therebetween in a composite.

(Method for Forming Polymer Film)

The method for forming a polymer film 3 comprises a step of bringing a polymer having a disulfide linkage in a side chain into contact with a metallic nanoparticle 2 to form a polymer film 3 in which the polymer is bonded to a surface of the metallic nanoparticle 2 with a sulfur atom interposed therebetween. The polymer as a raw material contains, for example, at least one group selected from the group consisting of a positively charged group 3b and a hydrophobic group 3c each bonded with a disulfide linkage interposed. In this case, in that step, at least one group selected from the group consisting of the positively charged group 3b and the hydrophobic group 3c is bonded to the surface of the metallic nanoparticle 2 together with the formation of the polymer film 3.

In the method for forming a polymer film 3, when a polymer having a positively charged group 3b and/or a hydrophobic group 3c in a side chain with a disulfide group interposing is used as a raw material, in the polymer film 3 to be formed, the polymer 3A to constitute the polymer film 3 may have a positively charged group 3b and/or a hydrophobic group 3c in a side chain with a disulfide group interposing. The positively charged group 3b and/or the hydrophobic group 3c in the polymer 3A is a group remaining without reacting in the method for forming the polymer film 3.

A concrete description will be made with reference to Fig. 6. Fig. 6 is a schematic view illustrating a method for forming a polymer film 3. The polymer 3B has, in a side chain, a positively charged group 3b and a hydrophobic group 3c each bonded with a disulfide linkage interposing. When the polymer 3B is brought into contact with the metallic nanoparticle 2, the bond between the sulfur atoms of each disulfide linkage is cleaved, the polymer 3B is bonded to the surface of the metallic nanoparticle 2 with a sulfur atom interposed therebetween, and in parallel, the positively charged group 3b and the hydrophobic group 3c are each bonded to the surface of the metallic nanoparticle 2 with a sulfur atom interposing. In the method for forming the polymer film 3, formation of the polymer film 3 on the surface of the metallic nanoparticle 2 and modification of the surface of the metallic nanoparticle 2 (for example, surface modification of the positively charged group 3b and/or the hydrophobic group 3c which are functional groups) can be performed in parallel in one step. Therefore, the present method is superior in cost.

(Specifically Bondable Substance)

The specifically bondable substance 4 is a nanosized (having a size of 3 to 15 nm at the longest) substance that is to be specifically bonded to a test substance (described in the fourth embodiment) in a specimen. Examples of the specifically bondable substance 4 include an antibody (hereinafter, referred to as a nanoantibody), a ligand, an enzyme, and a nucleic acid strand (more specifically, a DNA strand and an RNA strand). For example, the nanoantibody as the specifically bondable substance 4 is specifically bonded to an antigen as a test substance at the tip portion (antigen binding site) of the nanoantibody through an antigen-antibody reaction to form a composite. The ligand as the specifically bondable substance 4 forms a composite by specifically protein-ligand bonding to a protein as a test substance through a ligand receptor reaction. A nucleic acid strand as the specifically bondable substance 4 forms a pair (double strand) of a nucleic acid strand and another nucleic acid strand in a complementary relationship on the basis of the complementarity of a base pair. An enzyme as the specifically bondable substance 4 forms an enzyme-substrate composite with a substrate as a test substance on the basis of the substrate specificity (stereospecificity) at the active moiety (active center) of the enzyme. These specific bonds are non-covalent bonds, for example, hydrogen bonds and bonds caused by intermolecular force, hydrophobic interaction, and charge interaction.

Examples of the nanoantibody include variable domain of heavy chain antibody (VHH) antibodies, fragment antigen binding (Fab) antibodies, and variants thereof. A VHH antibody is a single domain antibody. The variant is an antibody in which a part of an amino acid sequence is recombined or an antibody in which a substituent is introduced, as long as the variant has specific binding to an antigen. The nanoantibody is preferably a VHH antibody. When the nanoantibody is a VHH antibody, because of a relatively small volume of the VHH antibody, it is possible to reduce the distance (separation distance) between two metallic nanoparticles 2 in a composite, more efficiently form a near field, and further increase the fluorescence intensity.

The molecular mass of the nanoantibody is preferably 60,000 Da or less, more preferably 30,000 Da or less, and still more preferably 20,000 Da or less. When the molecular mass is 60,000 Da or less (in particular, 30,000 Da or less, or 20,000 Da or less), since the volume of the nanoantibody is relatively small, it is possible to reduce the separation distance in a composite to efficiently form a near field and further increase the fluorescence intensity. Examples of the method for measuring the molecular mass include electrophoresis (SDS-PAGE), gel filtration chromatography, and static light scattering.

The specifically bondable substance 4 may be bonded directly to the polymer film 3, or may be bonded indirectly to the polymer film 3 with interposition of a linker portion (more specifically, $SM(PEG)_6$ or the like) derived from a crosslinker (more specifically, an NHS-maleimide group crosslinker or the like).

(Fluorescent Substance)

The fluorescent substance 6 is labeled on the surface of the polymer film 3 and/or the specifically bondable substance 4. The fluorescent substance 6 is excited by a near field formed by plasmon resonance and emits fluorescence. Examples of the fluorescent substance 6 include complexes of metals such as europium and ruthenium (metal complexes), and dyes of the Alexsa Fluor series (registered trademark) (manufactured by Molecular Probes).

The fluorescent substance 6 preferably has a large Stokes shift. Here, the Stokes shift is a difference between an absorption peak wavelength (maximum excitation wavelength) in an absorption spectrum of the fluorescent substance 6 and a fluorescence peak wavelength (maximum fluorescence wavelength) in a fluorescence spectrum. When the Stokes shift of the fluorescent substance 6 is large, the absorption spectrum and the fluorescence spectrum hardly overlap with each other and (scattered light of) excitation light hardly enters the fluorescence to be detected, so that more accurate fluorescence intensity can be measured.

Preferably, the fluorescence spectrum of the fluorescent substance is sharp. When the fluorescence spectrum is sharp, the fluorescence spectrum hardly overlaps with the absorption spectrum, and thus (scattered light of) excitation light hardly enters the fluorescence to be detected, so that fluorescence intensity can be measured more accurately.

(Measuring Device for Detecting Test Substance)

A measuring device using the above-described method for detecting a test substance will be described with reference to Fig. 13. Fig. 13 is a diagram illustrating a measuring device. As illustrated in Fig. 13, the measuring device 100 includes an excitation light source 110, an excitation light radiation optical system 120, a reagent container 130, a light receiving optical system 140, and a light receiving element 150.

The excitation light source 110 emits excitation light 112. The excitation light source 110 is, for example, a laser light source. The excitation light radiation optical system 120 performs adjustment of the sectional diameter like condensing of the excitation light 112, and outputs the incident excitation light 122. The excitation light radiation optical system 120 includes a lens 124 and a polarizing element ($\lambda/2$ plate) 126. The incident excitation light 122 output from the excitation light radiation optical system 120 is incident on the reagent container 130, and the measurement sample in the reagent container 130 is irradiated with the incident excitation light. The reagent container 130 is, for example, a detachable container (more specifically, a cell, a preparation, or the like) and a microchannel chip. The microchannel chip is a chip having a minute channel. When the reagent container 130 is a microchannel chip, for example, a nanoparticle body (reagent) according to the first embodiment and a specimen can be mixed and continuously supplied. Therefore, it is not necessary to prepare a measurement sample by mixing in advance, and it is possible to continuously perform measurement.

The measurement sample irradiated with the incident excitation light 122 emits fluorescence (detection light 132). The light receiving optical system 140 is arranged in a direction perpendicular to the traveling direction of the incident excitation light 122 toward the reagent container 130. The light receiving optical system 140 adjusts the sectional diameter or the like of the detection light 132 emitted from the measurement sample, and can remove scattered light of the incident excitation light 122 or adjust the quantity of light. The light receiving optical system 140 includes a lens 144 and an optical filter 146. The optical filter 146 includes, for example, a band pass filter and a dichroic mirror.

Fluorescence 142 having passed through light receiving optical system 140 is detected by the light receiving element 150. The light receiving element 150 includes, for example, a PD, an APD, a PMT, a CCD camera, and a spectroscope. The light receiving element 150 can measure the quantity of fluorescence of a single wavelength, measure a fluorescence spectrum, and create two-dimensional planar fluorescence imaging.

<Second Embodiment: Nanoparticle Body>

The second embodiment is different from the first embodiment in spectral characteristics of the fluorescent substance. This different configuration will be mainly described below. In the second embodiment, the same reference

numerals as those in the first embodiment have the same configurations as those in the first embodiment, and thus the description thereof will be omitted.

The fluorescence for detecting a test substance in the first embodiment was "excitation induced type fluorescence" induced by the overlap 208 of the absorption spectrum 202 of the fluorescent substance 6 with the plasmon resonance spectrum 206. The fluorescence for detecting a test substance in the second embodiment is "luminescence induced type fluorescence" induced by the overlap of the fluorescence spectrum of the fluorescent substance 6A with the plasmon resonance spectrum 206.

In the present description, the fluorescent substance in the nanoparticle body according to the second embodiment is also referred to as "fluorescent substance 6A" in order to indicate that the spectral characteristics thereof are different from those of the fluorescent substance 6 in the nanoparticle body according to the first embodiment and to distinguish from the fluorescent substance 6.

The nanoparticle body according to the present embodiment can enhance detection sensitivity. Without being bound by a particular theory, the reason for this is presumed as follows. In the nanoparticle body according to the present embodiment, a fluorescent substance 6A is excited by light having a luminous wavelength of plasmon resonance in a composite in which two or more nanoparticle bodies are bonded with a test substance interposed therebetween. As a result, the fluorescence spectrum of the fluorescent substance 6A and the plasmon resonance spectrum 206 derived from the metallic nanoparticle in the composite overlap with each other, and fluorescence for detecting the test substance is induced by a dipole-dipole mechanism due to multipole resonance, and the Purcell effect. Therefore, since the fluorescent substance 6A is sufficiently excited by light having a luminous wavelength of plasmon resonance derived from the composite, the detection sensitivity can be enhanced.

A description will be made with reference to Fig. 7. Fig. 7 is a conceptual diagram showing a plasmon resonance spectrum 201 derived from metallic nanoparticles 2 in a single particle state, a plasmon resonance spectrum 206 derived from metallic nanoparticles 2 in a composite, and a fluorescence spectrum 210 of a fluorescent substance 6A. In the present embodiment, the fluorescent substance 6A is selected such that there is a (preferably larger) overlap 212 of the fluorescence spectrum 210 of the fluorescent substance 6A with the resonance spectrum 206 derived from the metallic nanoparticle in the composite. As described above, when the fluorescence wavelength range $WR_E$ of the fluorescent substance 6A overlaps with the first luminous wavelength range $WR_{E1}$ of plasmon resonance, the fluorescent substance 6A is sufficiently excited and fluorescence is enhanced, so that detection sensitivity is greatly improved. For these reasons, it is considered that the nanoparticle body according to the present embodiment can enhance detection sensitivity.

In other words, from the viewpoint of enhancing the detection sensitivity for a test substance, it is important to select a fluorescent substance 6A such that the second region $R_2$ is made to exist. Here, the second region $R_2$ refers to a region where the multipole resonance spectrum 206 and the fluorescence spectrum 210 of the fluorescent substance overlap with each other (a region corresponding to the overlap 212) as illustrated in Fig. 7. From the viewpoint of further enhancing the detection sensitivity for a test substance, it is preferably important to select a fluorescent substance 6A such that the second region $R_2$ is made larger.

[Scheme 2]

The following is Scheme 2 of the process of detecting a test substance using the nanoparticle body according to the present embodiment:

[Chemical Formula 3]

(1) $\mathrm{Flu(S_0)} \xrightarrow{h\nu} \mathrm{Flu^*(S_1)}$ : Excitation of fluorescent substance

(2) $\mathrm{Flu^*(S_1)} \longrightarrow \mathrm{Flu(S_0)}$ + Fluorescence : Relaxation of exited fluorescent substance (Fluorescence emission)

(3) $\mathrm{M} \xrightarrow{h\nu\ (\text{Fluorescence})} \mathrm{M^*}$ + Resonant scattering light emission : Plasmon resonance (Multipole resonance)

$\mathrm{M^*} + \mathrm{Flu^*(S_1)} \xrightarrow{h\nu\ (\text{Fluorescence})} \mathrm{Flu(S_0)}$ + Fluorescence : Relaxation of exited fluorescent substance (Fluorescence emission)

in the elementary processes (1) to (3) of Scheme 2, $S_0$ denotes a ground state, $S_1$ denotes an excited singlet state, * denotes an excited state, Flu denotes a fluorescent substance 6A (in the composite), and M denotes a metallic nanoparticle 2 (in the composite).

Scheme 2 includes elementary processes (1) to (3).

As shown in the elementary process (1), for example, the fluorescent substance 6A is excited by external irradiation light.

As shown in the elementary process (2), the fluorescent substance 6A in the excited state relaxes and emits fluorescence.

As shown in the elementary process (3), plasmon resonance (multipole resonance) is induced on the surface of the metallic nanoparticle 2 by the emitted fluorescence (that is, plasmon resonance in the composite is induced by the fluorescence emitted by the fluorescent substance 6A). This plasmon resonance (multipole resonance) efficiently induces fluorescence emission of the fluorescent substance 6A in an excited state when the luminous wavelength range $WR_E$ of the fluorescent substance 6A overlaps with the first luminous wavelength range $WR_{E1}$ of the plasmon resonance.

In a preferred embodiment, the maximum fluorescence wavelength of the fluorescent substance 6A in the first luminous wavelength range $WR_{E1}$ is located at 500 to 700 nm (more preferably 550 to 700 nm, still more preferably 600 to 700 nm). That is, the maximum fluorescence wavelength of the fluorescent substance 6A is located at 500 to 700 nm in the first luminous wavelength region $WR_{E1}$ of the plasmon resonance spectrum derived from the metallic nanoparticle 2 in the composite. Examples of the fluorescent substance include fluorescein derivatives, rhodamine derivatives, cyanine dyes, and Alexa Flouor (registered trademark) manufactured by Molecular Probes. Among them, examples of the fluorescent substance 6A having a maximum fluorescence wavelength of 500 to 700 nm include "Ruthenium(II) tris(bipyridyl) -C5-NHS ester" manufactured by Tokyo Chemical Industry Co., Ltd. and Alexa Flour (registered trademark) series of 430, 488, 532, 546, 555, 568, 594, and 640. The maximum fluorescence wavelength can be determined as follows. An absorption spectrum of an aqueous solution of a fluorescent substance (solvent: deionized water) is measured, and a peak position of the absorption spectrum obtained is determined. The fluorescent substance 6A is irradiated with excitation light having the wavelength at the peak position, the fluorescence spectrum of the aqueous solution of the fluorescent substance is measured, and the peak position of the obtained fluorescence spectrum is defined as the maximum fluorescence wavelength.

<Third Embodiment: Nanoparticle Body>

The third embodiment is different from the first embodiment and the second embodiment in spectral characteristics of the fluorescent substance. This different configuration will be mainly described below. In the third embodiment, the same reference numerals as those in the first and second embodiments have the same configurations as those in the first and second embodiments, and thus the description thereof will be omitted.

In the third embodiment, the fluorescence for detecting a test substance is induced by the overlap 208 of the absorption spectrum 202 of the fluorescent substance 6 with the plasmon resonance spectrum 206 shown in the first embodiment, and is induced by the overlap 212 of the fluorescence spectrum 210 of the fluorescent substance 6A with the plasmon resonance spectrum 206 shown in the second embodiment. That is, the plasmon resonance in the composite is excitation induced type fluorescence induced by external irradiation light and also luminescence induced type fluorescence induced by fluorescence.

In the present description, the fluorescent substance in the nanoparticle body according to the third embodiment is also referred to as a "fluorescent substance 6B" in order to indicate that the spectral characteristics thereof are different from those of the fluorescent substance 6 in the nanoparticle body according to the first embodiment and those of the fluorescent substance 6A in the nanoparticle body according to the second embodiment and to distinguish from the fluorescent substance 6 and the fluorescent substance 6A.

A description will be made with reference to Fig. 8. Fig. 8 is a conceptual diagram showing a plasmon resonance spectrum 201 derived from metallic nanoparticles 2 in a single particle state, a plasmon resonance spectrum 206 of metallic nanoparticles 2 in a composite, and an absorption spectrum 202 and a fluorescence spectrum 210 of a fluorescent substance 6B. In the present embodiment, from the viewpoint of enhancing the detection sensitivity for a test substance, the fluorescent substance 6B is selected such that an overlap of the absorption spectrum 202 with the fluorescence spectrum 210 of the fluorescent substance 6B with the resonance spectrum 206 derived from the metallic nanoparticle 2 in the composite (an overlap 208 between the absorption wavelength range $WR_A$ and the first luminous wavelength range $WR_{E1}$ and an overlap 212 of the fluorescence wavelength range $WR_E$ with the first luminous wavelength range $WR_{E1}$, respectively) is allowed to exist. Furthermore, in a preferred embodiment, from the viewpoint of further enhancing the detection sensitivity for a test substance, the fluorescent substance 6B can be selected such that the overlap 208 and the overlap 212 are larger. Therefore, the fluorescent substance 6B is sufficiently excited and the fluorescence is enhanced, so that detection sensitivity is greatly improved. For these reasons, it is considered that the nanoparticle body according to the present embodiment can enhance detection sensitivity.

[Scheme 3]

The following is Scheme 3 of the process of detecting a test substance using the nanoparticle according to the present embodiment:

[Chemical Formula 4]

(1) $M \xrightarrow{h\nu \text{ (External irradiation light)}} M^*$ : Plasmon resonance (Multipole resonance)

(2) $M^* + Flu(S_0) \longrightarrow M + Flu^*(S_1) +$ Resonant scattering light emission
: Excitation of fluorescent substance

(3) $Flu^*(S_1) \longrightarrow Flu(S_0) +$ Fluorescence : Relaxation of exited fluorescent substance (Fluorescence emission)

(4) $M \xrightarrow{h\nu \text{ (Fluorescence)}} M^* +$ Resonant scattering light emission
: Plasmon resonance (Multipole resonance)

$M^* + Flu^*(S_1) \xrightarrow{h\nu \text{ (Fluorescence)}} Flu(S_0) +$ Fluorescence : Relaxation of exited fluorescent substance (Fluorescence emission)

in the elementary processes (1) to (4) of Scheme 3, $S_0$ denotes a ground state, $S_1$ denotes an excited singlet state, * denotes an excited state, Flu denotes a fluorescent substance 6B (in the composite), and M denotes a metallic nanoparticle 2 (in the composite).

Scheme 3 includes elementary processes (1) to (4).

As shown in the elementary process (1), when a composite formed by capturing a test substance is irradiated with external irradiation light, plasmon resonance (multipole resonance) is induced on the surface of the metallic nanoparticle 2 (that is, plasmon resonance in the composite is induced by external irradiation light.).

As shown in the elementary process (2), the fluorescent substance 6B is excited by a dipole-dipole mechanism and a near field. When the absorption wavelength range $WR_A$ of the fluorescent substance 6B overlaps with the first luminous wavelength range $WR_{E1}$ of plasmon resonance, the fluorescent substance 6B is efficiently excited.

As shown in the elementary process (3), the fluorescent substance 6B in the excited state relaxes and emits fluorescence.

As shown in the elementary process (4), plasmon resonance (multipole resonance) is induced on the surface of the metallic nanoparticle 2 by the emitted fluorescence (that is, plasmon resonance in the composite is induced by the fluorescence emitted by the fluorescent substance 6B). This plasmon resonance (multipole resonance) efficiently induces fluorescence emission of the fluorescent substance 6B in an excited state when the luminous wavelength range $WR_E$ of the fluorescent substance 6B overlaps with the first luminous wavelength range $WR_{E1}$ of the plasmon resonance.

The quantity of external irradiation light is larger than the quantity of fluorescence. Therefore, from the viewpoint of further improving the detection sensitivity, preferably, the fluorescent substance 6B is selected such that the first region $R_1$ in which the first luminous wavelength range $WR_{E1}$ and the absorption wavelength range $WR_A$ of the fluorescent substance 6B overlap with each other is larger than the second region $R_2$ in which the second luminous wavelength range $WR_{E2}$ and the absorption wavelength range $WR_A$ of the fluorescent substance 6B overlap with each other (see Fig. 8).

(Method for Determining Magnitude Relationship between first region $R_1$ and second region $R_2$)

The method for determining the magnitude relationship between the first region $R_1$ and the second region $R_2$ is performed as follows. The plasmon resonance spectrum of a composite is measured using a fluorescence microspectrometer (described in detail in Examples). A multipole resonance spectrum 206 in the plasmon resonance spectrum is identified. The absorption spectrum 202 and the fluorescence spectrum 210 of a fluorescent substance 6B are measured (the measurement sample of the fluorescent substance 6B is prepared with deionized water as a solvent).

An overlap 208 of a normalized multipole resonance spectrum 206 with a normalized absorption spectrum 202 of the fluorescent substance 6B is created. An integral value of the overlap 208 is calculated. An overlap 212 of a normalized multipole resonance spectrum 206 with a normalized fluorescence spectrum 210 of the fluorescent substance 6B is created. An integral value of the overlap 212 is calculated. On the basis of the obtained magnitude relationship of the integral value, the magnitude relationship between the first region $R_1$ and the second region $R_2$ is determined.

<Fourth Embodiment: Composite>

The composite will be described with reference to Fig. 9. Fig. 9 is a sectional view schematically illustrating the composite according to the fourth embodiment. The composite according to the fourth embodiment contains two nanoparticle bodies 1 according to the first embodiment, the two nanoparticle bodies 1 include a first nanoparticle body 10 and a second nanoparticle body 20, and the first nanoparticle body 10 and the second nanoparticle body 20 are bonded together with a test substance 30 interposed therebetween. The composite 40 comprises a test substance 30 to be detected and two nanoparticle bodies 10 and 20. In the composite 40, the two nanoparticle bodies 10 and 20 are bonded together with the test substance 30 interposed therebetween. That is, the nanoparticle bodies 10 and 20 according to the first embodiment form the composite according to the fourth embodiment bonded together with the test substance 30 interposing. Of the two nanoparticle bodies 10 and 20, one is referred to as a first nanoparticle body 10, and the other is referred to as a second nanoparticle body 20.

In the composite 40, the first nanoparticle body 10 includes the first metallic nanoparticle 12, the first polymer film 13 covering the surface of the first metallic nanoparticle 12, the first specifically bondable substance 14 bonded specifically to the test substance 30 in the specimen, and the first fluorescent substance 16 labeled on the first polymer film 13. The first specifically bondable substance 14 is bonded to the surface of the first polymer film 13. That is, the first nanoparticle body 10 includes the first metallic nanoparticle 12 as a metallic nanoparticle, the first polymer film 13 as a polymer film, the first specifically bondable substance 14 as a specifically bondable substance, and the first fluorescent substance 16 as a fluorescent substance.

In addition, in the composite 40, the second nanoparticle body 20 includes the second metallic nanoparticle 22, the second polymer film 23 covering the surface of the second metallic nanoparticle 22, the second specifically bondable substance 24 bonded specifically to the test substance 30 in the specimen, and the second fluorescent substance 26 labeled on the second polymer film 23. The second specifically bondable substance 24 is bonded to the surface of the second polymer film 23. That is, the second nanoparticle body 20 includes the second metallic nanoparticle 22 as a metallic nanoparticle, the second polymer film 23 as a polymer film, the second specifically bondable substance 24 as a specifically bondable substance, and the second fluorescent substance 26 as a fluorescent substance.

From the viewpoint of further enhancing the fluorescence intensity, the separation distance L is preferable to be as small as possible as long as excited fluorescent substances 16 and 26 are hardly quenched. More specifically, in a preferred embodiment, the two nanoparticle bodies 10 and 20 in the composite 40 are close to each other. In a more preferred embodiment, the two nanoparticle bodies 10, 20 are close to each other such that the first polymer film 13 of the first nanoparticle body 10 and the second polymer film 23 of the second nanoparticle body 20 in the composite 40 are in contact with each other. In a still more preferred embodiment, the two nanoparticle bodies 10 and 20 are close to each other in such a manner that the first polymer film 13 of the first nanoparticle body 10 and the second polymer film 23 of the second nanoparticle body 20 in the composite 40 come in contact with each other with at least one of the polymer films shrinking.

In a further preferred embodiment, in the case where the polymer films 13 and 23 come into contact with each other with at least one of the polymer films shrinking, for example, in the composite 40 illustrated in Fig. 9, it is considered to be possible to make at least one of the test substance 30, the specifically bondable substances 14 and 24 bonded to the test substance 30, and the fluorescent substances 16 and 26 to be embedded in the polymer films 13 and 23. In a still further preferred embodiment, in the case where the polymer films 13 and 23 are in contact with each other, for example, in the composite 40 illustrated in Fig. 9, it is considered to be possible, similarly to the further preferred embodiment described above, to make at least one of the test substance 30, the specifically bondable substances 14 and 24 that are bonded to the test substance 30, and the fluorescent substances 16 and 26 to be embedded in the polymer films 13 and 23 (the same applies to the composite illustrated in Fig. 10 described later).

In the present embodiment, since the films covering the surfaces of the metallic nanoparticles 12 and 22 are the polymer films 13 and 23, the fluorescence intensity can be enhanced. Without being bound by a particular theory, the reason for this is presumed as follows. The films covering the surfaces of the metallic nanoparticles 12 and 22 are the polymer films 13 and 23, and the polymer films 13 and 23 have relatively high flexibility as compared with an inorganic film containing an inorganic oxide. For this reason, in the composite 40, the polymer films 13 and 23 can shrink, so that the two metallic nanoparticles 12 and 22 can be closer to each other than a distance equivalent to a double of the thickness of each polymer film (the thickness of the polymer film 13 + the thickness of the polymer film 23). That is, since the films covering the surfaces of the metallic nanoparticles 12 and 22 are the polymer films 13 and 23, the separation distance L can be less than a double of the thickness of each polymer film. As a result, the plasmonic enhancement effect is easily obtained, and the fluorescence intensity is further enhanced. In the present description, the thickness of the polymer film in "a double of the thickness of each polymer film" is not the thickness of the polymer film 13 or 23 at the shrinking portion, which serves as the target of the separation distance, but is the thickness of the polymer film 13 or 23 at a non-shrinking portion, which does not serve as the target of the separation.

In the present embodiment, since the polymer films 13 and 23 each contain at least one selected from the group consisting of a binding site 3a with a sulfur atom interposing therein, a positively charged group 3b, and a hydrophobic

group 3c (for example, the polymer 3A constituting the polymer films 13 and 23 contains at least one selected from the group consisting of a binding site 3a with a sulfur atom interposing therein, a positively charged group 3b, and a hydrophobic group 3c), the fluorescence intensity can be further enhanced. Without being bound by a particular theory, the reason for this is presumed as follows. In such a case, at least one of the binding site 3a, the positively charged group 3b, and the hydrophobic group 3c forms a bond with the surfaces of the metallic nanoparticles 12 and 22. For this reason, it is considered that the polymer 3A has a network structure and covers the surfaces of the metallic nanoparticles 12 and 22 in a network manner. As described above, since the polymer 3A has a network structure, the polymer 3A further has relatively high flexibility. For this reason, in the composite 40, the polymer films 13 and 23 can further shrink, so that the two metallic nanoparticles 12 and 22 can be closer to each other than a distance equivalent to a double of the thickness of each polymer film. Therefore, in the present embodiment, the separation distance L can be less than a double of the thickness of each polymer film, the plasmonic enhancement effect is further obtained, and the fluorescence intensity is further enhanced.

In a preferred embodiment, the polymer 3A constituting the polymer films 13 and 23 contains at least one selected from the group consisting of a binding site 3a with a sulfur atom interposing therein, a positively charged group 3b, and a hydrophobic group 3c on a side chain (more specifically, at a side chain terminal) thereof. In a preferred embodiment, the fluorescence intensity can be further enhanced. Without being bound by a particular theory, the reason for this is presumed as follows. In such a case, at least one of the binding site 3a, the positively charged group 3b, and the hydrophobic group 3c forms a bond with the surfaces of the metallic nanoparticles 12 and 22. For this reason, it is considered that the polymer 3A has a network structure and covers the surfaces of the metallic nanoparticles 12 and 22 in a network manner with side chains thereof used as binding sites. As described above, since the polymer 3A has a network structure, the polymer 3A further has relatively high flexibility. For this reason, in the composite 40, the polymer films 13 and 23 can further shrink, so that the two metallic nanoparticles 12 and 22 can be closer to each other than a distance equivalent to a double of the thickness of each polymer film. Therefore, in the present embodiment, the separation distance L can be less than a double of the thickness of each polymer film, the plasmonic enhancement effect is further obtained, and the fluorescence intensity is further enhanced.

The separation distance L between the first nanoparticle body 10 and the second nanoparticle body 20 is, for example, 12 nm to 52 nm, and preferably 12 nm to 27 nm. The separation distance L is a distance between the first metallic nanoparticle 12 and the second metallic nanoparticle 22, and is a distance with which a line segment connecting between a first point P1 on the surface of the first nanoparticle body 10 and a second point P2 on the surface of the second nanoparticle body 20 is minimized. In a case where the separation distance L is 52 nm or less, when the composite 40 is irradiated with excitation light, a near field occurs more efficiently in a space near the surfaces between the first and second metallic nanoparticles 12 and 22, so that the fluorescence intensity can be further enhanced.

The films covering the surfaces of the metallic nanoparticles 12 and 22 are polymer films 13 and 23, the polymer 3A constituting the polymer films 13 and 23 has at least one selected from the group consisting of a binding site 3a with a sulfur atom interposing therein, a positively charged group 3b, and a hydrophobic group 3c (for example, on a side chain (more specifically, at a side chain terminal)). Therefore, as described above, the separation distance L can be smaller than the distance equivalent to a double of the thickness of each polymer film covering the surfaces of the two metallic nanoparticles 12 and 22 in the composite 40. For example, when the polymer films 13 and 23 have a thickness of 5 nm, the separation distance L can be less than 10 nm (more specifically, 2 to 9 nm, 3 to 8 nm, 4 to 7 nm, or the like).

The composite 40 may include two nanoparticle bodies 10 and 20 as illustrated in Figs. 9 to 11, and may include three nanoparticle bodies 10, 20 and 60 as illustrated in Fig. 12, for example. The composite may be composed by containing four or more nanoparticle bodies. Fig. 11 is a diagram illustrating a composite 40 composed of two nanoparticle bodies. Fig. 12 is a diagram illustrating a composite 40 composed of three nanoparticle bodies.

In the first to third embodiments, it has been described that the multipole resonance is plasmon resonance derived from the metallic nanoparticles 12 and 22 in the composite 40. A description in more detail will be made with reference to Figs. 11 and 12. In Fig. 11, the binding site (more specifically, the test substance 30 and the specifically bondable substances 14 and 24) between the nanoparticle bodies 10 and 20 in the composite 40 is represented by a binding point 50 (the same applies to Fig. 12). In the composite 40 composed of the two nanoparticle bodies 10 and 20, when the oscillation direction of the electric field component of excitation light (polarization direction of the electric field component of the excitation light) is parallel to the long-axis direction $D_{LA}$ of the composite 40, free electrons on the surfaces of the metallic nanoparticles 12 and 22 can cause collective oscillation in the long-axis direction $D_{LA}$. Thus, multipole resonance is caused by a dipole-dipole interaction. On the other hand, when the oscillation direction of the electric field component of the excitation light is parallel to the short-axis direction $D_{SA}$ of the composite 40, free electrons on the surfaces of the metallic nanoparticles 12 and 22 may cause collective oscillation in the short-axis direction $D_{SA}$. For this reason, dipole resonance is caused similarly to the metallic nanoparticles in a single particle state.

In the composite 40 composed of three nanoparticle bodies 10, 20, and 60, the number of oscillation directions $D_1$, $D_2$, and $D_3$ of the electric field component of the excitation light allowed for multipole resonance is 3, which is larger than that of the composite 40 composed of the two nanoparticle bodies 10 and 20. Furthermore, the number of the binding

points 50 of the composite 40 composed of the three nanoparticle bodies 10, 20, and 60 is 3, which is larger than that of the composite 40 composed of the two nanoparticle bodies 10 and 20. In this manner, a plurality of binding points 50 (where two nanoparticle bodies 10 and 20 are bonded with one test substance 30 interposed therebetween) may be included. Therefore, as the number of the nanoparticle bodies 10 and 20 constituting the composite 40 increases, fluorescence is more likely to be enhanced, and there is a possibility that the composite is superior in detection sensitivity.

(Fluorescent Substance)

As illustrated in Figs. 9 and 10, the fluorescent substances 16 and 26 are preferably positioned between the first metallic nanoparticle 12 and the second metallic nanoparticle 22. This is because, since the place between the metallic nanoparticles 12 and 22 is a space where the near field is efficiently generated, the fluorescence intensity is easily enhanced by the Purcell effect due to positioning the fluorescent substances 16 and 26 in the space between the metallic nanoparticles 12 and 22.

(Test Substance)

The test substance 30 is a substance to be detected contained in a specimen. Examples of the test substance 30 include an antigen, a protein, a substrate, and a nucleic acid strand. The test substance 30 is specifically bonded to the specifically bondable substances 14 and 24. For example, an antigen has at least two epitopes and forms specific bonding with the first and second specifically bondable substances 14 and 24 at the epitopes. Examples of the antigen include proteins such as c-reactive protein, myoglobin, troponin T, troponin I, and BNP, and antigen proteins of viruses such as influenza virus and RS virus. The test substance 30 is a test substance derived from a specimen such as blood, plasma, urine, or saliva. That is, examples of the specimen containing the test substance 30 include blood, plasma, serum, urine, and saliva. The specimen may further contain a solvent and a buffer (more specifically, phosphate-buffered saline (PBS), Tris buffer, HEPES buffer, MOPS buffer, MES buffer, or the like).

<Other Embodiments>

The present invention is not limited to the above-described embodiments, and can be modified in design without departing from the gist of the present invention.

In the first embodiment, the polymer 3B has one positively charged group 3b and one hydrophobic group 3c each bonded with a disulfide linkage interposed, but the present invention is not limited to this configuration. The polymer 3B may have two or more positively charged groups 3b and two or more hydrophobic groups 3c each independently. That is, in the polymer 3B, each of the number of side chains to which the positively charged group 3b is bonded and the number of side chains to which the hydrophobic group 3c is bonded may be 2 or more.

In the fourth embodiment, the first and second fluorescent substances 16 and 26 are labeled on the first and second polymer films 13 and 23, respectively, but the present invention is not limited to this configuration. For example, Fig. 10 is a sectional view schematically illustrating a composite according to a modification example of the fourth embodiment. As illustrated in Fig. 10, the first and second fluorescent substances 16 and 26 may be labeled on the first and second specifically bondable substances 14 and 24, respectively. This case is more preferable because the first and second fluorescent substances 16 and 26 are easily positioned between the first metallic nanoparticle 12 and the second metallic nanoparticle 22 and the detection intensity is improved. One of the first and second fluorescent substances 16 and 26 may be labeled on the polymer films 13 and 23, and the other may be labeled on the specifically bondable substances 14 and 24.

In the fourth embodiment, two fluorescent substances 16 and 26 are labeled on the composite 40, but the present invention is not limited to this configuration. For example, the number of the fluorescent substances labeled on the composite 40 may be 1 or 3 or more.

In the fifth embodiment, the light receiving optical system 140 in the measuring device 100 is disposed in a direction perpendicular to the traveling direction of the incident excitation light 122 toward the reagent container 130, but the present invention is not limited to this configuration. For example, the light receiving optical system 140 may be arranged in a direction parallel to the traveling direction of the incident excitation light 122, or may be arranged in a direction having an acute angle or an obtuse angle with respect to the traveling direction of the incident excitation light 122.

Examples

Hereinafter, the present invention will be described in more detail with reference to Examples. The present invention is not limited by the following Examples at all. In addition, unless otherwise specified, parts and % in the Examples are on mass basis. Examples and Comparative Examples were performed in the air and at normal temperature (1 atm, 25°C) unless otherwise specified.

In Examples and Comparative Examples, the concentration of a metallic nanoparticle in a dispersion may be expressed by absorbance. The absorbance was measured using an ultraviolet-visible spectrophotometer ("infinite M200 PRO" manufactured by TECAN Japan Co., Ltd.). Since the absorption wavelength varies depending on each sample, the absorption wavelength was described for each sample. Note that a subscript number attached to the notation OD of the absorbance indicates an absorption wavelength. For example, "$OD_{455}$ = 0.1" indicates that the absorbance at a wavelength of 455 nm is 0.1.

<Example 1>

[1. Formation of Polymer Film]

The method for forming a polymer film will be described with reference to Figs. 14 and 15. Figs. 14 and 15 are schematic diagrams showing the method for forming the polymer film of Example 1. As shown in Fig. 14, poly-L-lysine ("3075" manufactured by Peptide Institute, Inc.) and 3-(2-pyridyldithio)propionamide-$PEG_4$-NHS (manufactured by Thermo Fisher SCIENTIFIC Inc., serial number "26128", "NHS-$PEG_4$-SPDP") were stirred and mixed under the conditions of at room temperature for 4 hours using a small rotary incubator ("RT-30 mini" manufactured by TAITEC Corporation). As a result, a polymer was obtained. This synthesis reaction is a nucleophilic substitution reaction in which a primary amino group of poly-L-lysine attacks the NHS ester group of 3-(2-pyridyldithio)propionamide-$PEG_4$-NHS. The polymer 3B synthesized had a disulfide linkage in a side chain. More specifically, the polymer synthesized had a hydrophobic group (pyridyl group) 3c and a positively charged group (primary ammonium group) 3b each bonded with a disulfide linkage interposed.

The polymer 3B obtained was brought into contact with a metallic nanoparticle 2 to form a polymer film 3. More specifically, the polymer 3B obtained was added to 1 mL of a dispersion of a silver nanoparticle ("AGCB80-1M" manufactured by nanoComposix, diameter: 80 nm, $OD_{455}$ = 0.1) as the metallic nanoparticle 2, and the mixture was stirred and mixed under the conditions of at room temperature overnight using a small rotary incubator ("RT-30 mini" manufactured by TAITEC Corporation). As a result, a dispersion of the silver nanoparticle covered with the polymer film was obtained.

As illustrated in Fig. 15, the polymer film 3 contains, on the surface of the silver nanoparticle 2, a binding site 3a with a sulfur atom interposing therein, a hydrophobic group (pyridyl group) 3c that forms a hydrophobic bond with the surface of the silver nanoparticle 2, and a positively charged group (primary ammonium group) 3b that forms an electrostatic bond b with the surface of the silver nanoparticle 2. That is, the polymer 3A constituting the polymer film 3 has, on the surface of the silver nanoparticle 2, a binding site 3a with a sulfur atom interposing therein, a hydrophobic group (pyridyl group) 3c that forms a hydrophobic bond c with the surface of the silver nanoparticle 2, and a positively charged group (primary ammonium group) 3b that forms an electrostatic bond with the surface of the silver nanoparticle 2. An SEM image (500,000 magnifications) of the silver nanoparticle 2 obtained was prepared, and it was confirmed that the surface of the silver nanoparticle 2 was continuously covered with the polymer film 3 (hereinafter, the silver nanoparticle covered with the polymer film 3 is referred to as "polymer-covered silver nanoparticle"). In addition, the thickness of the polymer film 3 covering the silver nanoparticle 2 was measured from the SEM image.

[2. Preparation of Nanoparticle Body]

Fig. 16 is a schematic diagram illustrating a structure of a nanoparticle body 1 to which a fluorescently labeled antibody of Example 1 is bonded. The nanoparticle body illustrated in Fig. 16 was prepared by first bonding a crosslinker to the surface of a polymer-coated metallic nanoparticle, separately bonding a fluorescent substance and a crosslinker to a nanoantibody, and then bonding the crosslinker bonded to the polymer-coated metallic nanoparticle and the crosslinker bonded to the nanoantibody. Hereinafter, details of the preparation of a nanoparticle body to which a fluorescently labeled antibody is bonded will be described.

(2-1. Bonding of Crosslinker to Polymer Film-Coated Silver Nanoparticle)

To 1 mL of the prepared dispersion of a polymer film-coated silver nanoparticle were further added a crosslinker SM(PEG)2 (PEGylated, long-chain SMCC crosslinker) ("22105" manufactured by Thermo Fisher SCIENTIFIC Inc.) and sodium heparin ("081-00136 " manufactured by FUJIFILM Wako Pure Chemical Corporation), and the mixture was stirred and mixed under the conditions of at room temperature for 1 hour using a small rotary incubator ("RT-30 mini" manufactured by TAITEC Corporation). As a result, a dispersion of a silver nanoparticle covered with a polymer film 3 to which a crosslinker SM(PEG)2 is bonded (hereinafter, also referred to as a polymer film-coated silver nanoparticles to which an SM(PEG)2 linker is bonded) was obtained. The SM(PEG)2 linker bonded to the polymer film-coated silver nanoparticle had a maleimide group.

(2-2. Fluorescently Labeling to VHH Antibody)

An NHS-labeled Ru complex derivative ("Ruthenium(II) tris(bipyridyl)-C5-NHS ester" manufactured by Tokyo Chemical Industry Co., Ltd.) represented by the chemical formula of [Chemical Formula 5]:

[Chemical Formula 5]

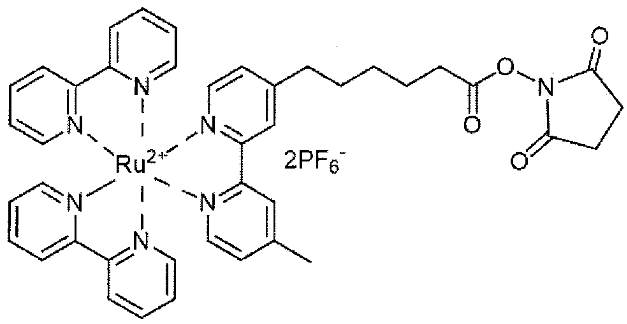

was added to 100 μg of a VHH antibody (manufactured by RePHAGEN Co., Ltd., molecular mass: 18,000 Da), and the mixture was stirred and mixed under the conditions of at room temperature for 1 hour using a small rotary incubator ("RT-30 mini" manufactured by TAITEC Corporation). As a result, a VHH antibody with a fluorescent substance bonded thereto (hereinafter, also referred to as a fluorescently labeled VHH antibody) was obtained. The NHS-labeled Ru complex derivative is a fluorescent substance having a maximum fluorescence wavelength at 500 to 700 nm.

(2-3. Bonding of Crosslinker to Fluorescently Labeled VHH Antibody)

Subsequently, 3-(2-pyridyldithio)propionamide-PEG4-NHS (manufactured by Thermo Fisher SCIENTIFIC Inc., product number "26128", "NHS-PEG4-SPDP") as an NHS-bipyridyl disulfide crosslinker was added to the fluorescent substance-labeled VHH antibody in a molar ratio of 8 times equivalent, and the mixture was stirred and mixed under the conditions of at room temperature for 1 hour using a small rotary incubator ("RT-30 mini" manufactured by TAITEC Corporation). As a result, a VHH antibody to which a fluorescent substance and an SPDP linker were bonded (hereinafter, also referred to as a fluorescently labeled VHH antibody with an SPDP linker bonded thereto) was obtained.

(2-4. Thiolation of Fluorescently Labeled VHH Antibody with Crosslinker Bonded Thereto)

Next, to the fluorescently labeled VHH antibody with an SPDP linker bonded thereto, a reducing agent TCEP ("77720" manufactured by Thermo Fisher SCIENTIFIC Inc.) was added in a molar ratio of 2 times equivalent, and the mixture was stirred and mixed under the conditions of at 37°C for 1 hour using a stirrer ("TS-100" manufactured by BioSan). As a result, a VHH antibody to which a fluorescent substance and an SPDP linker having been reduced (hereinafter, also referred to as a reduced SPDP linker) were bonded (hereinafter, also referred to as a fluorescently labeled VHH antibody with a reduced SPDP linker bonded thereto) was obtained. The reduced SPDP linker had a thiol group (-SH group) generated through reduction of a disulfide linkage.

(2-5. Bonding of Fluorescently Labeled VHH Antibody to Silver Nanoparticle)

Subsequently, the fluorescently labeled VHH antibody with a reduced SPDP linker bonded thereto was added to a dispersion ($OD_{430}$ = 0.1) of a polymer film-coated silver nanoparticle with a maleimide group-containing SM(PEG)6 linker bonded thereto, and the mixture was stirred and mixed under the conditions of at room temperature overnight using a small rotary incubator ("RT-30 mini" manufactured by TAITEC Corporation). As a result, the maleimide group of the SM(PEG)6 linker reacted with the thiol group of the reduced SPDP linker, affording a nanoparticle body to which a fluorescently labeled VHH antibody was bonded with a linker moiety interposing (see Fig. 16).

[3. Preparation of Composite]

C Reactive Protein ("00-AGN-AP-CRP-00" manufactured by ADVY CHEMICAL Sigma-Aldrich Co. LLC) (hereinafter, also referred to as a CRP antigen) as a test substance 30 was added to a phosphate buffer solution of the resulting nanoparticle body, and the mixture was stirred under the conditions of at room temperature for 5 minutes using a small

rotary incubator ("RT-30 mini" manufactured by TAITEC Corporation) to prepare a measurement sample ($OD_{455}$ = 0.4). As a blank, a phosphate buffer solution of a nanoparticle body containing no CRP antigen 30 was separately prepared.

[4. Evaluation Method]

(4-1. Detection of test substance by immunochromatography)

Fig. 17 is a schematic diagram illustrating an immunochromatography strip (hereinafter, also simply referred to as "strip"). The strip 300 has a strip shape, and includes a sample pad 301 which is disposed at one end of the strip and to which a measurement sample is dropped, and a determination line 303 disposed at the center of the strip. On the determination line 303 is immobilized a CPR-antigen as a test substance 30. When the measurement sample is dropped on the sample pad 301, the composite 40 in the measurement sample moves toward the determination line 303 (along the direction 305) due to a capillary phenomenon. When the composite 40 reaches the determination line 303, an antigen-antibody reaction with the CRP antigen occurs, and the composite 40 is captured.

A measurement sample was dropped on the sample pad 301 of the strip 300 and left at rest for a prescribed time. The strip 300 left at rest was set in a fluorometer ("In-house experimental fluorometer" manufactured by PHC Corporation). The determination line 303 was irradiated with excitation light (wavelength: 415 to 455 nm), and the quantity of fluorescence (detection wavelength: 573 nm or 613 nm) was measured. The measurement value of the blank was subtracted from the obtained measurement value to determine the presence or absence of the quantity of fluorescence derived from the fluorescent substance. As a result, in the preparation of the measurement sample, fluorescence was detected when the concentration of the CPR-antigen was 42 pM (unit: $\times 10^{-12}$ mol/L) or more.

The strip 300 left at rest was set in an absorptiometer ("In-house experimental absorptiometer" manufactured by PHC Corporation). The determination line 303 was irradiated with visible light (wavelength: 415 to 455 nm) to measure reflected light. The measurement value of the blank was subtracted from the obtained measurement value to determine the presence or absence of reflected light derived from the metallic nanoparticles. As a result, in the preparation of the measurement sample, reflected light was detected when the concentration of the CRP antigen was 670 pM or more.

The CRP antigen as the test substance 30 was detected by immunography. This confirmed that the composite 40 was formed in the measurement sample and that the composite 40 was present in the determination line.

(4-2. Analysis of Plasmon Resonance Wavelength in Presence of Test Substance)

First, a sample for measurement was prepared. The measurement sample was prepared in the same manner as 4-1. The measurement sample was dropped on a slide glass, and a glass cover plate was placed on the droplet to sandwich the droplet, thereby preparing a preparation for measurement as a sample for measurement. Note that the preparation for measurement was used for measurement of a plasmon resonance spectrum in a state where the droplet was present.

A preparation for blank was prepared in the same manner as in the preparation for measurement except that the CRP antigen was not added.

Next, the plasmon resonance spectrum of the measurement target was measured using a fluorescence microspectrometer. The measurement target (composite) on the preparation for measurement was continuously irradiated with excitation light (wavelength: 400 to 650 nm) finely narrowed, and a plasmon resonance spectrum was measured. The measurement target (composite) of Example 1 was a composite constituted by bonding two nanoparticle bodies in a diatomic molecular form with a test substance (CRP antigen) interposed therebetween. The measurement result is shown in Fig. 18(b). Fig. 18(b) is a diagram showing a plasmon resonance spectrum of Example 1 for the preparation for measurement as a sample for measurement.

The preparation for blank was measured in the same manner. In this measurement, a nanoparticle body in a monodispersed state (primary particle state) was used as a measurement target. The measurement result is shown in Fig. 18(a). Fig. 18(a) is a diagram showing a plasmon resonance spectrum of Example 1 for the preparation for blank as a sample for measurement.

(Measurement Results)

As shown in Fig. 18(a), the plasmon resonance spectrum of the preparation for blank had a spectral shape having a single peak near 460 to 470 nm. This peak was assigned to plasmon resonance (dipole resonance) caused by monodispersed Ag nanoparticles.

As shown in Fig. 18(b), the plasmon resonance spectrum of the preparation for measurement showed a spectral shape having a peak near 460 to 470 nm (peak on a shorter wavelength side) and a peak near 590 to 600 nm (peak on a longer wavelength side). The peak on the shorter wavelength side was assigned to plasmon resonance (dipole

resonance) induced by excitation light having a polarization direction of an electric field component parallel to the short-axis direction of the composite (sandwich type composite including two nanoparticle bodies). The peak on the longer wavelength side was assigned to plasmon resonance (multipole resonance) induced by excitation light having a polarization direction of an electric field component parallel to the long-axis direction of the composite.

Separately, an absorption spectrum and a fluorescence spectrum of a fluorescent substance (Ru complex "Ruthenium(II) tris(bipyridyl)-C5-NHS ester" manufactured by Tokyo Chemical Industry Co., Ltd.) were measured using a spectrophotometer ("infinite M200 PRO" manufactured by TECAN Japan Co., Ltd.). The absorption spectrum was measured at an arbitrary concentration in an aqueous solvent at a measurement wavelength of 230 to 500 nm. The measurement conditions of the fluorescence spectrum were an excitation wavelength of 450 nm, a measurement wavelength of 500 to 700 nm, and the same measurement sample as the absorption spectrum. Fig. 19 was created by superimposing the absorption spectrum and the fluorescence spectrum of the obtained fluorescent substance on the plasmon resonance spectrum of the preparation for measurement of Fig. 18(b).

Fig. 19 is a diagram illustrating a plasmon resonance spectrum of Example 1, and an absorption spectrum 202 and a fluorescence spectrum 210 of a Ru complex. As shown in Fig. 19, an overlap 212 (second region $R_2$) of the fluorescence wavelength range $WR_E$ of the fluorescence spectrum 210 of the Ru complex with the first luminous wavelength range $WR_{E1}$ of the peak on the longer wavelength side (plasmon resonance spectrum 206) of the plasmon resonance spectrum was observed. Therefore, it was concluded that the fluorescence detected in the system of Example 1 was luminescence induced type fluorescence.

<Example 2>

[3. Preparation of Nanoparticle Body]

In the preparation of the nanoparticle body of Example 2, a nanoparticle body was prepared in the same manner as in Example 1 except that the metallic nanoparticle was changed from 1 mL of a dispersion of a silver nanoparticle "AGCB80-1M" manufactured by nanoComposix, diameter: 80 nm, $OD_{455}$ = 0.1 to a silver nanoparticle "AGCB50-1M" manufactured by nanoComposix, diameter: 50 nm, $OD_{455}$ = 0.1.

(4-2. Analysis of Plasmon Resonance Wavelength in Presence of Test Substance)

The plasmon resonance wavelength in the presence of a test substance was analyzed in the same manner as in Example 1. The measurement results are shown in Fig. 20. Fig. 20 is a diagram showing plasmon resonance spectra of Example 2 for (a) the preparation for blank and (b) the preparation for measurement. The measurement target in Example 2 was a composite composed of 15 nanoparticle bodies.

As shown in Fig. 20(a), the plasmon resonance spectrum of the preparation for blank had a spectral shape having a single peak near 460 to 470 nm. This peak was assigned to plasmon resonance (dipole resonance) caused by monodispersed Ag nanoparticles.

As shown in Fig. 20(b), the plasmon resonance spectrum of the preparation for measurement showed a spectral shape having a peak near 460 to 470 nm (peak on the shorter wavelength side) and a peak near 590 to 600 nm (peak on the longer wavelength side). The peak on the shorter wavelength side was assigned to plasmon resonance (dipole resonance) induced by excitation light having a polarization direction of an electric field component parallel to the short-axis direction of the composite. The peak on the longer wavelength side was assigned to plasmon resonance (multipole resonance) induced by excitation light having a polarization direction of an electric field component parallel to the long-axis direction of the composite.

Fig. 21 was created by superimposing the absorption spectrum and the fluorescence spectrum of the Ru complex obtained in Example 1 on the plasmon resonance spectrum of the preparation for measurement of Fig. 20(b). As shown in Fig. 21, an overlap 212 (second region $R_2$) of the fluorescence wavelength range $WR_E$ of the fluorescence spectrum 210 of the Ru complex with the first luminous wavelength range $WR_{E1}$ of the peak on the longer wavelength side (plasmon resonance spectrum 206) of the plasmon resonance spectrum was observed. Therefore, it was concluded that the fluorescence detected in the system of Example 2 was luminescence induced type fluorescence.

<Example 3>

[3. Preparation of Nanoparticle Body]

In the preparation of the nanoparticle body of Example 3, a nanoparticle body was prepared in the same manner as in Example 1 except that the fluorescent substance was changed from the NHS-labeled Ru complex derivative ("Ruthenium(II) tris(bipyridyl)-C5-NHS ester" manufactured by Tokyo Chemical Industry Co., Ltd.) to Alexa Fluor 594

carboxylic acid, succinimidyl ester ("A10169" manufactured by Invitrogen). The Alexa Fluor 594 is a fluorescent substance having a maximum absorption wavelength and a maximum fluorescence wavelength at 500 to 700 nm.

(4-1. Detection of test substance by immunochromatography)

In the same manner as in Example 1, a test substance was detected by immunochromatography. The wavelength of the excitation light with which the determination line 303 was irradiated was 600 nm. As a result of measuring the quantity of fluorescence, in the preparation of the measurement sample, fluorescence was detected when the concentration of the CPR antigen was 42 pM or more. As a result of measuring reflected light, in the preparation of the measurement sample, reflected light was detected when the concentration of the CPR antigen was 670 pM or more. The CRP antigen as the test substance was detected by immunography. This confirmed that the composite 40 was formed in the measurement sample and that the composite 40 was present in the determination line.

(4-2. Analysis of Plasmon Resonance Wavelength in Presence of Test Substance)

Separately, an absorption spectrum and a fluorescence spectrum of a fluorescent substance (Alexa Fluor 594 carboxylic acid, succinimidyl ester ("A10169" 20004 manufactured by Invitrogen)) were measured using a spectrophotometer ("infinite M200 PRO" manufactured by TECAN Japan Co., Ltd.). The measurement conditions of the absorption spectrum include a measurement wavelength of 450 to 650 nm, and the solvent of the measurement sample was deionized water.

Fig. 22 was created by superimposing the absorption spectrum and the fluorescence spectrum of the obtained fluorescent substance on the plasmon resonance spectrum of the preparation for measurement of Fig. 20(b). Fig. 22 is a diagram illustrating an absorption spectrum and a fluorescence spectrum of a fluorescent substance of Example 3, and a plasmon resonance spectrum of Example 1. The fluorescence spectrum 210 of the fluorescent substance of Example 3 has a peak near 584 nm. The fluorescence spectrum 210 of the fluorescent substance of Example 3 has a peak near 613 nm.

The plasmon resonance spectrum of the system of Example 1 was adopted as an alternative to the system of Example 3. This is because the composite of Example 3 differs from the composite of Example 1 only in the type of the fluorescent substance, but the material and particle size of the metallic nanoparticle that mainly characterize the plasmon resonance spectrum are the same (the plasmon resonance spectrum slightly includes the absorption of the fluorescent substance, and therefore this point is considered to be different).

As shown in Fig. 22, an overlap 208 (first region $R_1$) of the absorption wavelength range $WR_A$ of the absorption spectrum 202 of the fluorescent substance with the first luminous wavelength range $WR_{E1}$ of the peak on the longer wavelength side (multipole resonance spectrum) 206 of the plasmon resonance spectrum was observed. Therefore, it was concluded that the fluorescence detected in the system of Example 3 was mainly excitation induced type fluorescence.

(4-3. Preparation of Composite)

A CRP antigen as a test substance 30 was added to the phosphate buffer solution of the obtained nanoparticle body, and a reaction (antigen-antibody reaction) was performed under conditions of room temperature and 10 minutes at a final concentration of 90 ng/mL. A mixed solution of the composite composed of the nanoparticle body and the test substance was thereby prepared. The mixed liquid prepared was used as a measurement sample. As a blank, a phosphate buffer solution of a nanoparticle body containing no CRP antigen 30 was separately prepared.

(4-4. Fluorescence Analysis of Test Substance-Nanoparticle Body System)

The test substance-nanoparticle body system was subjected to fluorescence analysis. Using a spectrofluorometer ("FP-8550" manufactured by JASCO Corporation), a fluorescence spectrum after composite formation was measured under the conditions of an excitation wavelength of 580 nm and a measurement wavelength range of 600 to 720 nm. The measured results are shown in Fig. 23. Fig. 23 is a diagram showing a fluorescence spectrum of the test substance-nanoparticle body system. The solid line indicates the test substance-nanoparticle body system, and the broken line indicates the fluorescence spectrum of a blank sample. The fluorescence spectrum of the test substance-nanoparticle body system has a peak near 614 nm. The intensity of the peak near 614 nm is more than about 4 times the intensity of the peak near 614 nm in the fluorescence spectrum of the blank sample.

As shown in Fig. 22, the fluorescent substance of Example 3 has a maximum absorption wavelength near 586 nm. The absorption spectrum 202 of the fluorescent substance of Example 3 largely overlaps with the multipole resonance spectrum 206. It was confirmed that fluorescence was effectively enhanced as shown in Fig. 23 by using a fluorescent substance having an absorption matching the light of the plasmon resonance wavelength of the composite as shown in

Fig. 22.

<Comparative Example 1>

A silver nanoparticle covered with silica (manufactured by nanoComposix, particle diameter of silver nanoparticle (core particle diameter): 50 nm, thickness of silica film: 20 nm) was diluted with water (deionized water) to prepare an aqueous dispersion of a silver nanoparticle covered with silica. The resulting aqueous dispersion was used as a measurement sample. Specifically, in the measurement sample, an agglomerate in which two or more silver nanoparticles were agglomerated was also present in addition to the primary particle state of the silver nanoparticle covered with silica (hereinafter also referred to as "silica-covered silver nanoparticle").

The plasmon resonance wavelength in the presence of a test substance was analyzed in the same manner as in Example 1. The measurement results are shown in Fig. 24. Fig. 24 shows a plasmon resonance spectrum of Comparative Example 1 ((a) silica-covered silver nanoparticles in a primary particle state and (b) an aggregate in which three silica-covered silver nanoparticles are arranged in a substantially linear form). In Fig. 24(a), silica-covered silver nanoparticles in a primary particle state are used as a measurement target, and in Fig. 24(b), an aggregate in which three silica-covered silver nanoparticles are arranged in a substantially linear form is used as a measurement target. The plasmon resonance spectrum shown in Fig. 24(a) exhibited a spectral shape having one peak assigned to dipole resonance near 450 nm. The plasmon resonance spectrum shown in Fig. 24(b) exhibited a spectral shape having one peak assigned to dipole resonance near 470 nm similarly to Fig. 24(a). In Comparative Example 1, no peak of the plasmon resonance spectrum caused by a multipole was observed.

<Comparative Example 2>

A silver nanoparticle covered with silica (manufactured by nanoComposix, particle diameter of silver nanoparticle (core particle diameter): 50 nm, thickness of silica film: 7 nm) was diluted with water (deionized water) to prepare an aqueous dispersion of a silver nanoparticle covered with silica. The resulting aqueous dispersion was used as a measurement sample. Specifically, in the measurement sample, an agglomerate in which two or more silver nanoparticles were agglomerated was also present in addition to the primary particle state of the silver nanoparticle covered with silica.

The plasmon resonance wavelength in the presence of a test substance was analyzed in the same manner as in Example 1. The measurement results are shown in Fig. 25. Fig. 25 shows a plasmon resonance spectrum of Comparative Example 2 ((a) silica-covered silver nanoparticles in a primary particle state and (b) an aggregate in which three silica-covered silver nanoparticles are arranged in a substantially ozone molecule shape). In Fig. 25(a), silica-covered silver nanoparticles in a primary particle state are used as a measurement target, and in Fig. 25(b), an aggregate in which three silica-covered silver nanoparticles are arranged in a substantially ozone molecule shape is used as a measurement target. The plasmon resonance spectrum shown in Fig. 25(a) exhibited a spectral shape having one peak assigned to dipole resonance near 450 nm. The plasmon resonance spectrum shown in Fig. 25(b) exhibited a spectral shape having one peak assigned to dipole resonance near 450 nm similarly to Fig. 25(a). In Comparative Example 2, no peak of the plasmon resonance spectrum caused by a multipole was observed.

Embodiments of the nanoparticle body, the composite containing a nanoparticle body, and the method for forming a polymer film contained in a nanoparticle body according to the present disclosure are as follows.

<1> A nanoparticle body comprising:

a metallic nanoparticle;
a polymer film covering a surface of the metallic nanoparticle;
a specifically bondable substance that specifically bonds to a test substance in a specimen; and
a fluorescent substance labeled on a surface of the polymer film or on the specifically bondable substance, wherein
the fluorescent substance is excited by light having a luminous wavelength of plasmon resonance in a composite in which two or more of the nanoparticle body are bonded together with the test substance interposed therebetween.

<2> The nanoparticle body according to <1>, wherein the plasmon resonance in the composite is induced by light applied from outside.
<3> The nanoparticle body according to <1> or <2>, wherein the plasmon resonance in the composite is induced by fluorescence emitted by the fluorescent substance.
<4> The nanoparticle body according to any one of <1> to <3>, wherein an absorption wavelength range of the fluorescent substance overlaps with a first luminous wavelength range of the plasmon resonance.

<5> The nanoparticle body according to any one of <1> to <4>, wherein a first luminous wavelength range of the plasmon resonance is located on a longer wavelength side as compared to a second luminous wavelength range of the plasmon resonance induced in a single particle of the metallic nanoparticle.
<6> The nanoparticle body according to <5>, wherein the fluorescent substance is selected such that a first region where the first luminous wavelength range and an absorption wavelength range of the fluorescent substance overlap with each other is larger than a second region where the second luminous wavelength range and the absorption wavelength range of the fluorescent substance overlap with each other.
<7> The nanoparticle body according to <5> or <6>, wherein a maximum absorption wavelength of the fluorescent substance in the first luminous wavelength range is located at 500 to 700 nm.
<8> The nanoparticle body according to any one of <5> to <7>, wherein a maximum fluorescence wavelength of the fluorescent substance in the first luminous wavelength range is located at 500 to 700 nm.
<9> The nanoparticle body according to any one of <1> to <8>, wherein the plasmon resonance induced in the composite is multipole resonance.
<10> The nanoparticle body according to any one of <5> to <8>, wherein the plasmon resonance induced in the metallic nanoparticles as the single particle is dipole resonance.
<11> The nanoparticle body according to any one of <1> to <10>, wherein the polymer film contains at least one selected from the group consisting of a binding site with a sulfur atom interposing therein, a positively charged group, and a hydrophobic group, between the polymer film and a surface of the metallic nanoparticle.
<12> The nanoparticle body according to <11>, wherein the polymer film contains at least the positively charged group in a side chain of a polymer constituting the polymer film, and
the positively charged group is at least one selected from the group consisting of a primary ammonium group, a secondary ammonium group, a tertiary ammonium group, a quaternary ammonium group, and a guanidyl group ($-NHC(=NH_2^+)NH_2$).
<13> The nanoparticle body according to <11>, wherein the polymer film contains at least the hydrophobic group in a side chain of a polymer constituting the polymer film, and
the hydrophobic group is at least one selected from the group consisting of an aromatic cyclic group, an aliphatic cyclic group, and an aliphatic chain group.
<14> The nanoparticle body according to any one of <1> to <13>, wherein the polymer film has a thickness of 1 nm to 10 nm.
<15> The nanoparticle body according to any one of <1> to <14>, which is a nanoparticle body to be used for plasmon-excited fluorescence analysis.
<16> The nanoparticle body according to any one of <1> to <15>, wherein the specifically bondable substance is a nanoantibody.
<17> The nanoparticle body according to any one of <1> to <16>, wherein the specifically bondable substance is a VHH antibody.
<18> The nanoparticle body according to any one of <1> to <17>, wherein the metallic nanoparticle comprises gold or silver.
<19> The nanoparticle body according to any one of <1> to <18>, wherein the metallic nanoparticle has a particle diameter of 5 to 100 nm.
<20> The nanoparticle body according to any one of <1> to <19>, wherein

a first nanoparticle body and a second nanoparticle body are contained as the nanoparticle body, and
the first nanoparticle body and the second nanoparticle body form a composite in which the first nanoparticle body and the second nanoparticle body are bonded together with a test substance interposed therebetween.

<21> The nanoparticle body according to any one of <1> to <20>, wherein the test substance is a test substance derived from the specimen that is blood, plasma, urine, or saliva.
<22> The nanoparticle body according to <20>, wherein in the composite, the fluorescent substance is positioned between the first nanoparticle body and the second nanoparticle body.
<23> A composite comprising two or more of the nanoparticle body according to any one of <1> to <22>, wherein the two or more nanoparticle bodies include a first nanoparticle body and a second nanoparticle body, and the first nanoparticle body and the second nanoparticle body are bonded together with the test substance interposed therebetween.
<24> The composite according to <23>, wherein the composite contains a plurality of binding points at which two of the nanoparticle body are bonded together with the test substance interposed therebetween.
<25> A method for forming a polymer film, comprising a step of bringing a polymer having a disulfide linkage in a side chain into contact with a metallic nanoparticle to form a polymer film in which the polymer is bonded to a surface of the metallic nanoparticle with a sulfur atom interposed therebetween.

<26> The method for forming a polymer film according to <25>, wherein

the polymer contains at least one group selected from the group consisting of a positively charged group and a hydrophobic group each bonded with the disulfide linkage interposed, and
in the step, at least one group selected from the group consisting of the positively charged group and the hydrophobic group is bonded to a surface of the metallic nanoparticle together with the formation of the polymer film.

REFERENCE SIGNS LIST

| | |
|---|---|
| 1 | Nanoparticle body |
| 2 | Metallic nanoparticle |
| 3 | Polymer film |
| 3A | Polymer constituting polymer film |
| 3B | Polymer forming polymer film |
| 3a | Binding site with a sulfur atom interposing therein |
| 3b | Positively charged group |
| 3c | Hydrophobic group |
| 4 | Specifically bondable substance |
| 6 | Fluorescent substance |
| 10 | First nanoparticle body |
| 12 | First metallic nanoparticle |
| 13 | First polymer film |
| 14 | First specifically bondable substance |
| 16 | First fluorescent substance |
| 20 | Second nanoparticle body |
| 22 | Second metallic nanoparticle |
| 23 | Second polymer film |
| 24 | Second specifically bondable substance |
| 26 | Second fluorescent substance |
| 30 | Test substance |
| 40 | Composite |
| L | Separation distance (separative distance) |
| $R_1$ | First region |
| $R_2$ | Second region |
| $WR_A$ | Absorption wavelength range of fluorescent substance |
| $WR_E$ | Luminous wavelength range of fluorescent substance |
| $WR_{E1}$ | First luminous wavelength range |
| $WR_{E2}$ | Second luminous wavelength range |

**Claims**

**1.** A nanoparticle body comprising:

a metallic nanoparticle;
a polymer film covering a surface of the metallic nanoparticle;
a specifically bondable substance that specifically bonds to a test substance in a specimen; and
a fluorescent substance labeled on a surface of the polymer film or on the specifically bondable substance, wherein
the fluorescent substance is excited by light having a luminous wavelength of plasmon resonance in a composite in which two or more of the nanoparticle body are bonded with the test substance interposed therebetween.

**2.** The nanoparticle body according to claim 1, wherein the plasmon resonance in the composite is induced by light applied from outside.

**3.** The nanoparticle body according to claim 1 or 2, wherein the plasmon resonance in the composite is induced by

fluorescence emitted by the fluorescent substance.

4. The nanoparticle body according to any one of claims 1 to 3, wherein an absorption wavelength range of the fluorescent substance overlaps with a first luminous wavelength range of the plasmon resonance.

5. The nanoparticle body according to any one of claims 1 to 4, wherein a first luminous wavelength range of the plasmon resonance is located on a longer wavelength side as compared to a second luminous wavelength range of the plasmon resonance induced in a single particle of the metallic nanoparticle.

6. The nanoparticle body according to claim 5, wherein the fluorescent substance is selected such that a first region where the first luminous wavelength range and an absorption wavelength range of the fluorescent substance overlap with each other is larger than a second region where the second luminous wavelength range and the absorption wavelength range of the fluorescent substance overlap with each other.

7. The nanoparticle body according to claim 5 or 6, wherein a maximum absorption wavelength of the fluorescent substance in the first luminous wavelength range is located at 500 to 700 nm.

8. The nanoparticle body according to any one of claims 5 to 7, wherein a maximum fluorescence wavelength of the fluorescent substance in the first luminous wavelength range is located at 500 to 700 nm.

9. The nanoparticle body according to any one of claims 1 to 8, wherein the plasmon resonance induced in the composite is multipole resonance.

10. The nanoparticle body according to any one of claims 5 to 8, wherein the plasmon resonance induced in the metallic nanoparticles as the single particle is dipole resonance.

11. The nanoparticle body according to any one of claims 1 to 10, wherein the polymer film contains at least one selected from the group consisting of a binding site with a sulfur atom interposing therein, a positively charged group, and a hydrophobic group, between the polymer film and a surface of the metallic nanoparticle.

12. The nanoparticle body according to claim 11, wherein the polymer film contains at least the positively charged group in a side chain of a polymer constituting the polymer film, and
the positively charged group is at least one selected from the group consisting of a primary ammonium group, a secondary ammonium group, a tertiary ammonium group, a quaternary ammonium group, and a guanidyl group ($-NHC(=NH_2^+)NH_2$).

13. The nanoparticle body according to claim 11, wherein the polymer film contains at least the hydrophobic group in a side chain of a polymer constituting the polymer film, and
the hydrophobic group is at least one selected from the group consisting of an aromatic cyclic group, an aliphatic cyclic group, and an aliphatic chain group.

14. The nanoparticle body according to any one of claims 1 to 13, wherein the polymer film has a thickness of 1 nm to 10 nm.

15. The nanoparticle body according to any one of claims 1 to 14, which is a nanoparticle body to be used for plasmon-excited fluorescence analysis.

16. The nanoparticle body according to any one of claims 1 to 15, wherein the specifically bondable substance is a nanoantibody.

17. The nanoparticle body according to any one of claims 1 to 16, wherein the specifically bondable substance is a VHH antibody.

18. The nanoparticle body according to any one of claims 1 to 17, wherein the metallic nanoparticle comprise gold or silver.

19. The nanoparticle body according to any one of claims 1 to 18, wherein the metallic nanoparticle has a particle diameter of 5 to 100 nm.

**20.** The nanoparticle body according to any one of claims 1 to 19, wherein

a first nanoparticle body and a second nanoparticle body are contained as the nanoparticle body, and
the first nanoparticle body and the second nanoparticle body form a composite in which the first nanoparticle body and the second nanoparticle body are bonded together with a test substance interposed therebetween.

**21.** The nanoparticle body according to any one of claims 1 to 20, wherein the test substance is a test substance derived from the specimen that is blood, plasma, urine, or saliva.

**22.** The nanoparticle body according to claim 20, wherein in the composite, the fluorescent substance is positioned between the first nanoparticle body and the second nanoparticle body.

**23.** A composite comprising two or more of the nanoparticle body according to any one of claims 1 to 22, wherein the two or more nanoparticle bodies include a first nanoparticle body and a second nanoparticle body, and the first nanoparticle body and the second nanoparticle body are bonded together with the test substance interposed therebetween.

**24.** The composite according to claim 23, wherein the composite contains a plurality of binding points at which two of the nanoparticle body are bonded together with the test substance interposed therebetween.

**25.** A method for forming a polymer film, comprising a step of bringing a polymer having a disulfide linkage in a side chain into contact with a metallic nanoparticle to form a polymer film in which the polymer is bonded to a surface of the metallic nanoparticle with a sulfur atom interposed therebetween.

**26.** The method for forming a polymer film according to claim 25, wherein

the polymer contains at least one group selected from the group consisting of a positively charged group and a hydrophobic group each bonded with the disulfide linkage interposed therebetween, and
in the step, at least one group selected from the group consisting of the positively charged group and the hydrophobic group is bonded to a surface of the metallic nanoparticle together with the formation of the polymer film.

*Fig.1*

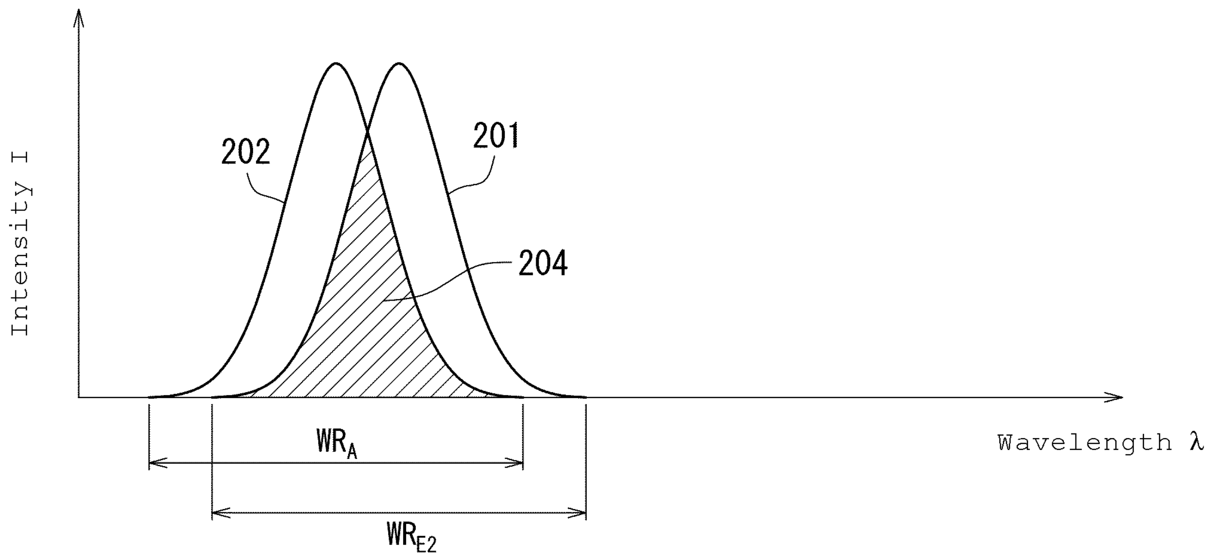
Intensity I
202
201
204
Wavelength λ
WRA
WRE2

*Fig.2*

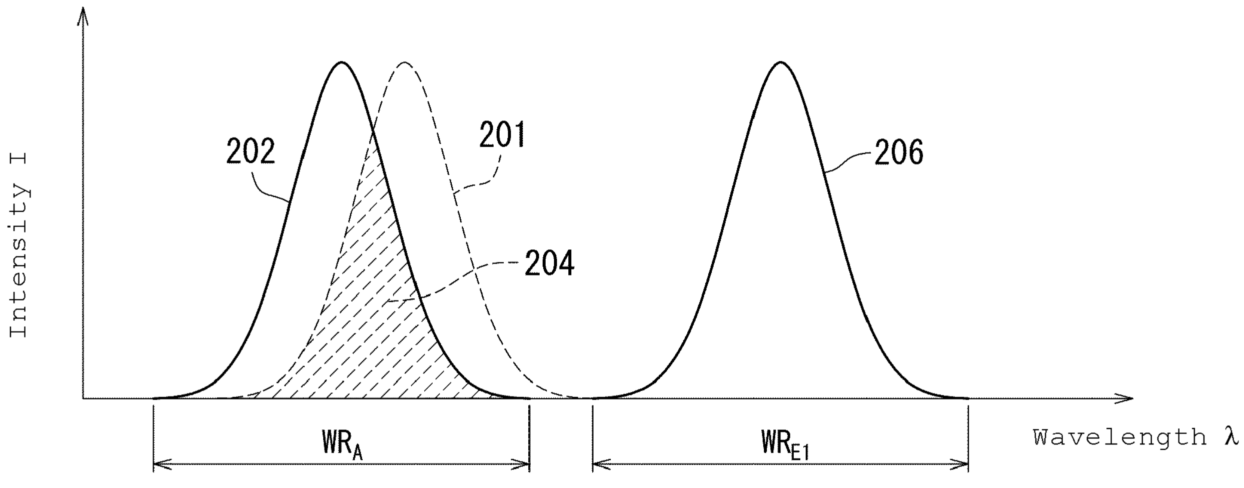
Intensity I
202
201
204
206
Wavelength λ
WRA
WRE1

Fig.3

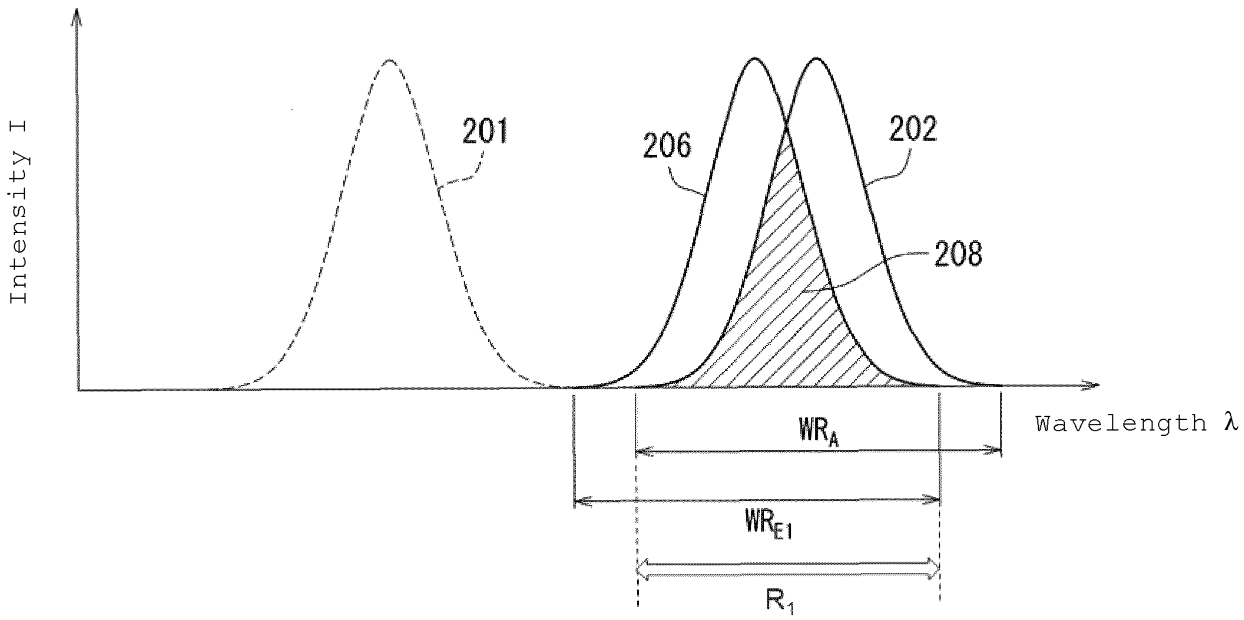


Fig.4

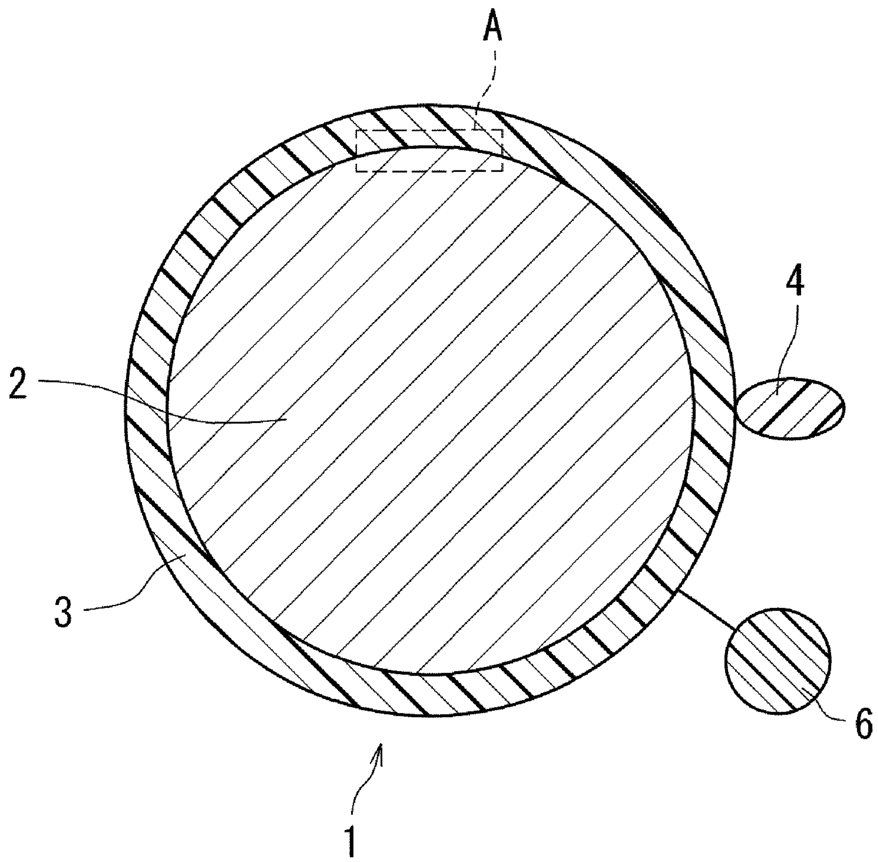

*Fig.5*

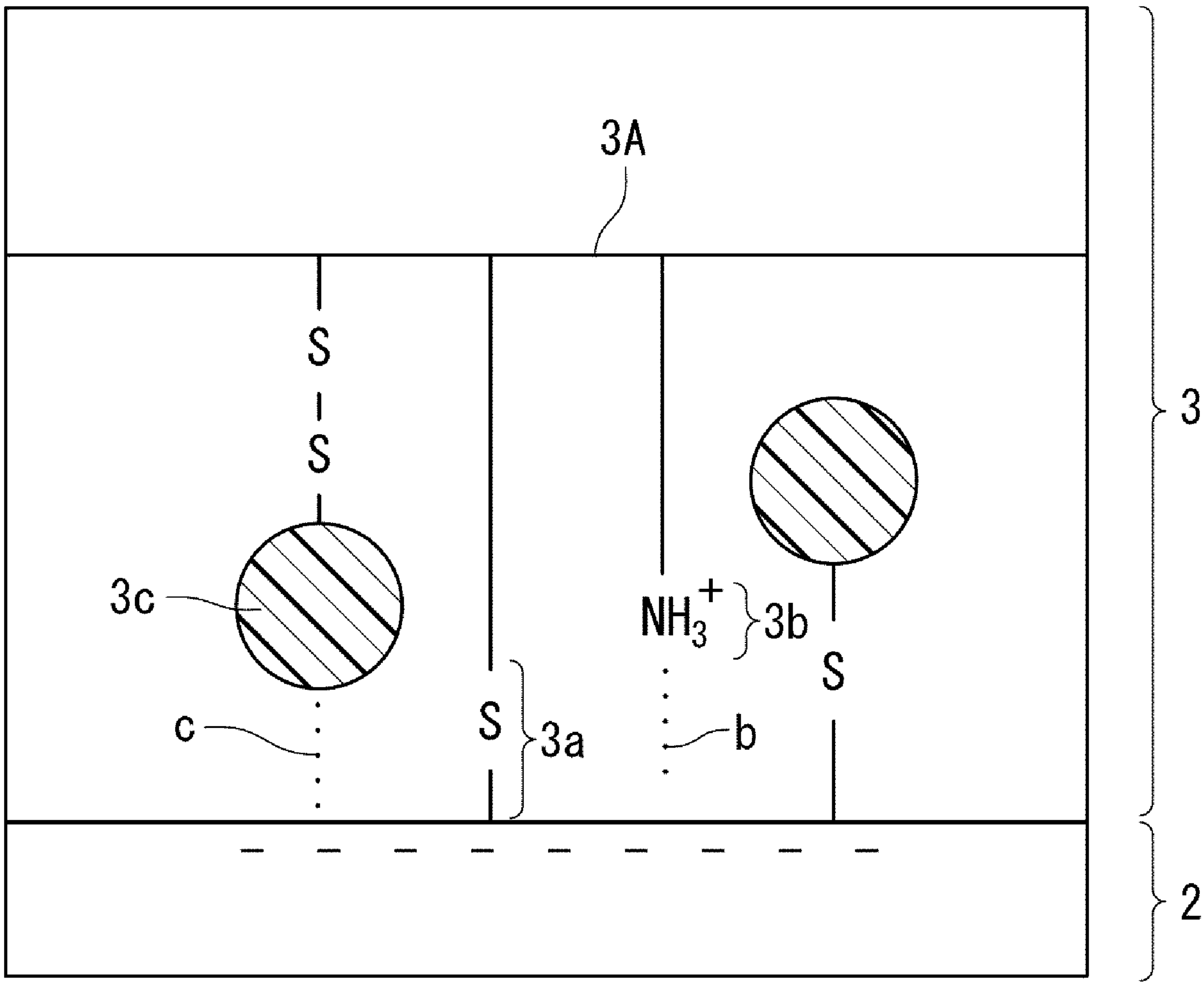

*Fig.6*

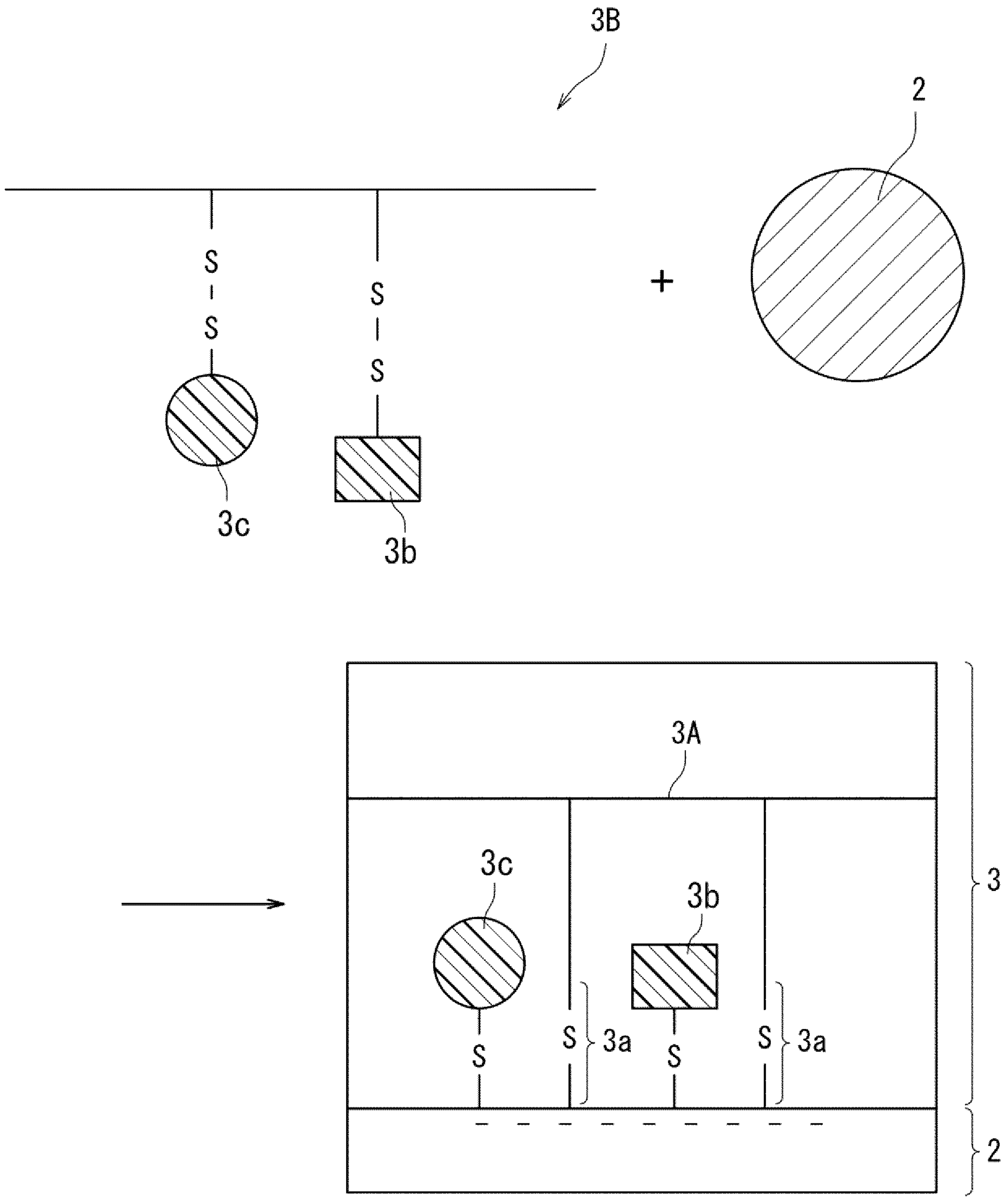

*Fig.7*

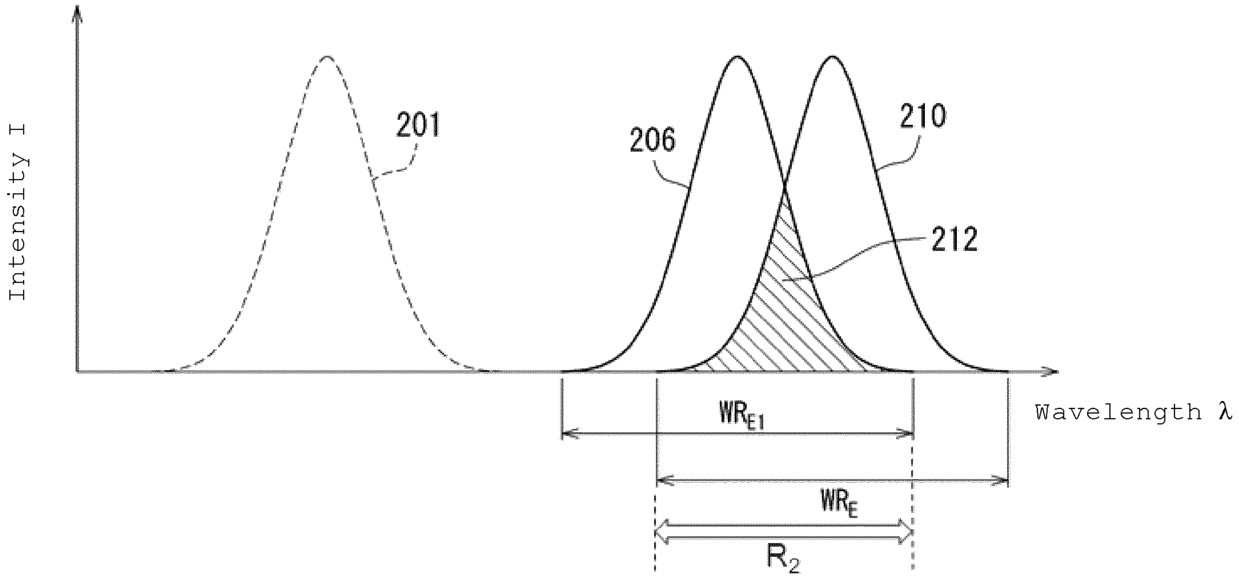
Intensity I
201
206
210
212
Wavelength λ
WR_E1
WR_E
R_2

*Fig.8*

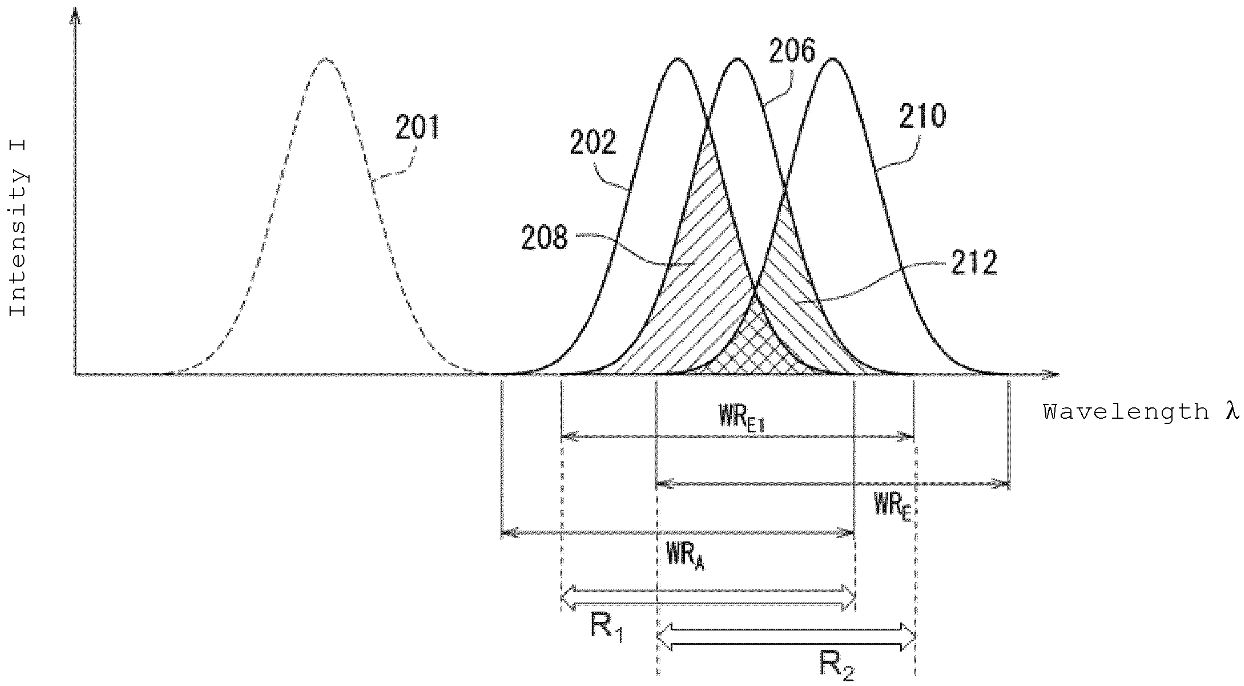
Intensity I
201
202
206
208
210
212
Wavelength λ
WR_E1
WR_E
WR_A
R_1
R_2

Fig.9

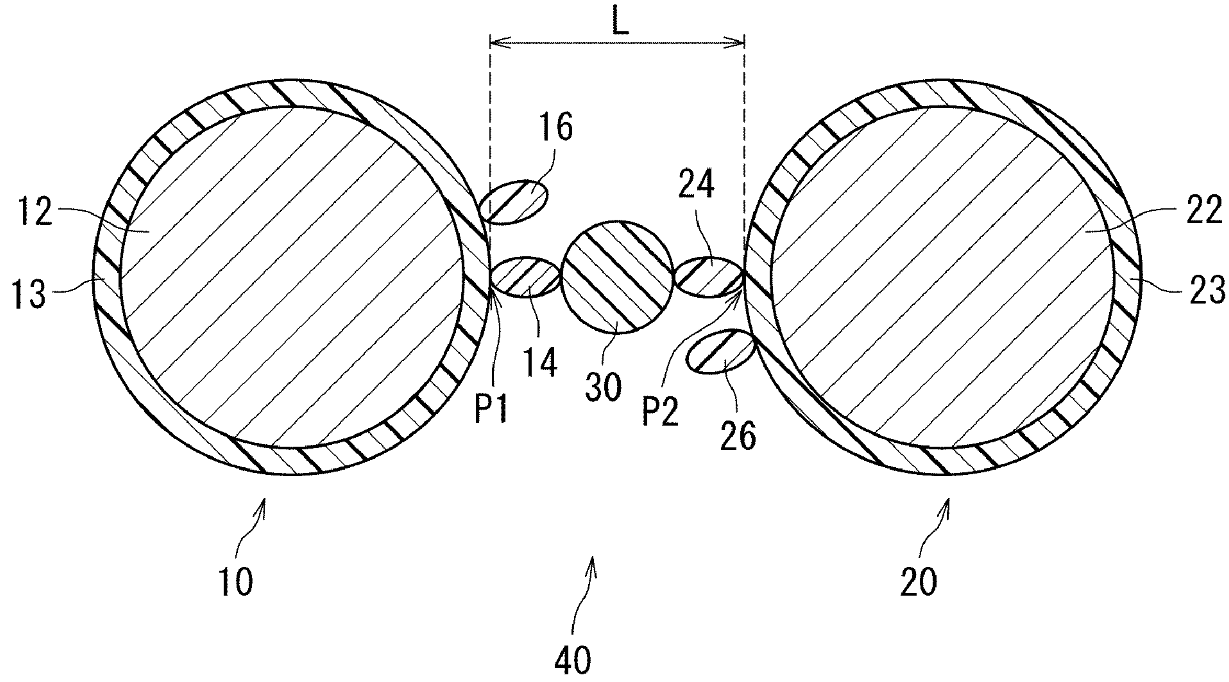
L
16
24
12
22
13
23
14
30
P1
P2
26
10
20
40

Fig.10

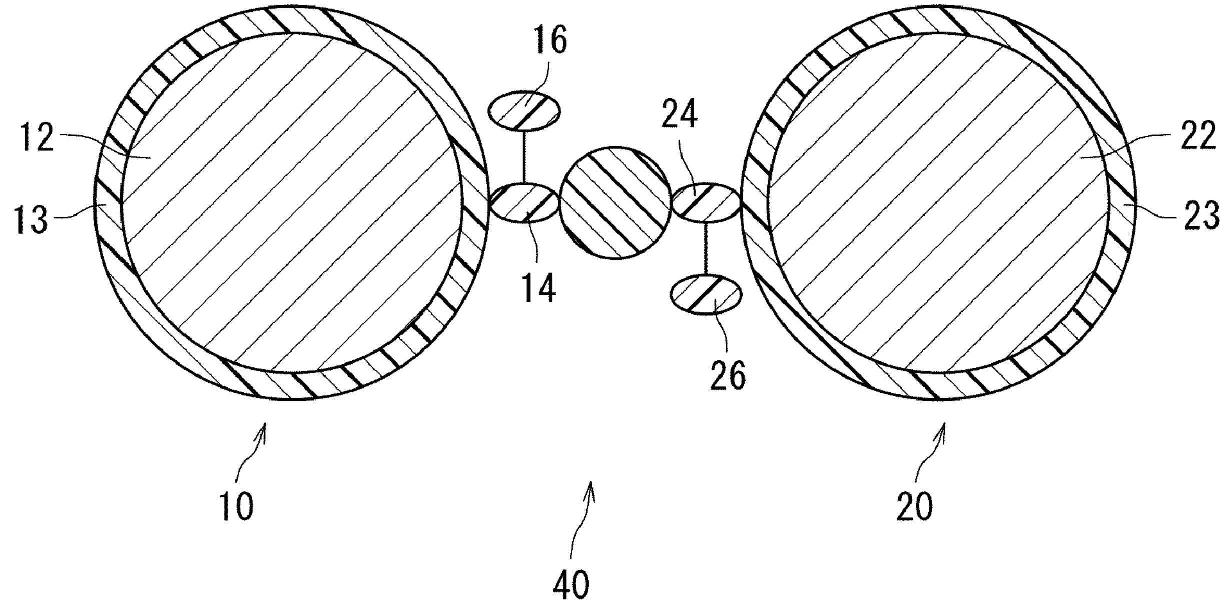
16
24
12
22
13
23
14
26
10
20
40

Fig.11

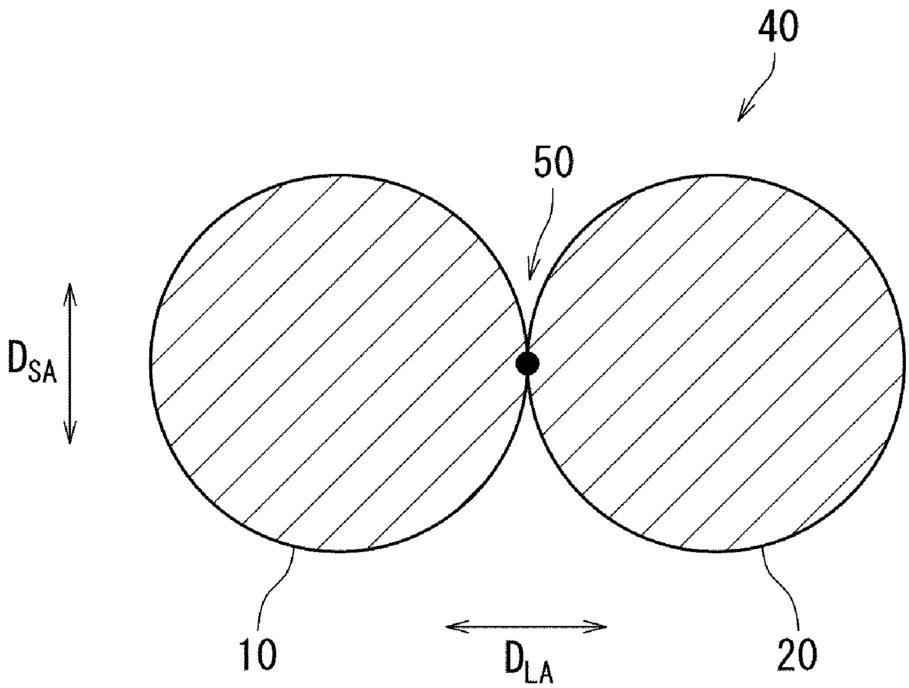


Fig.12

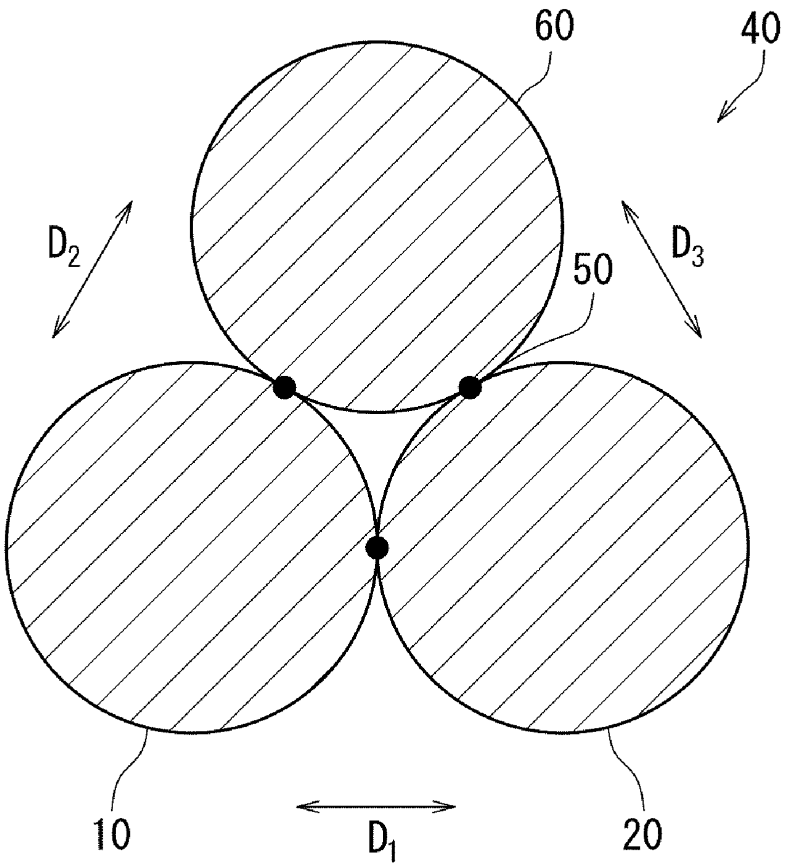

Fig.13

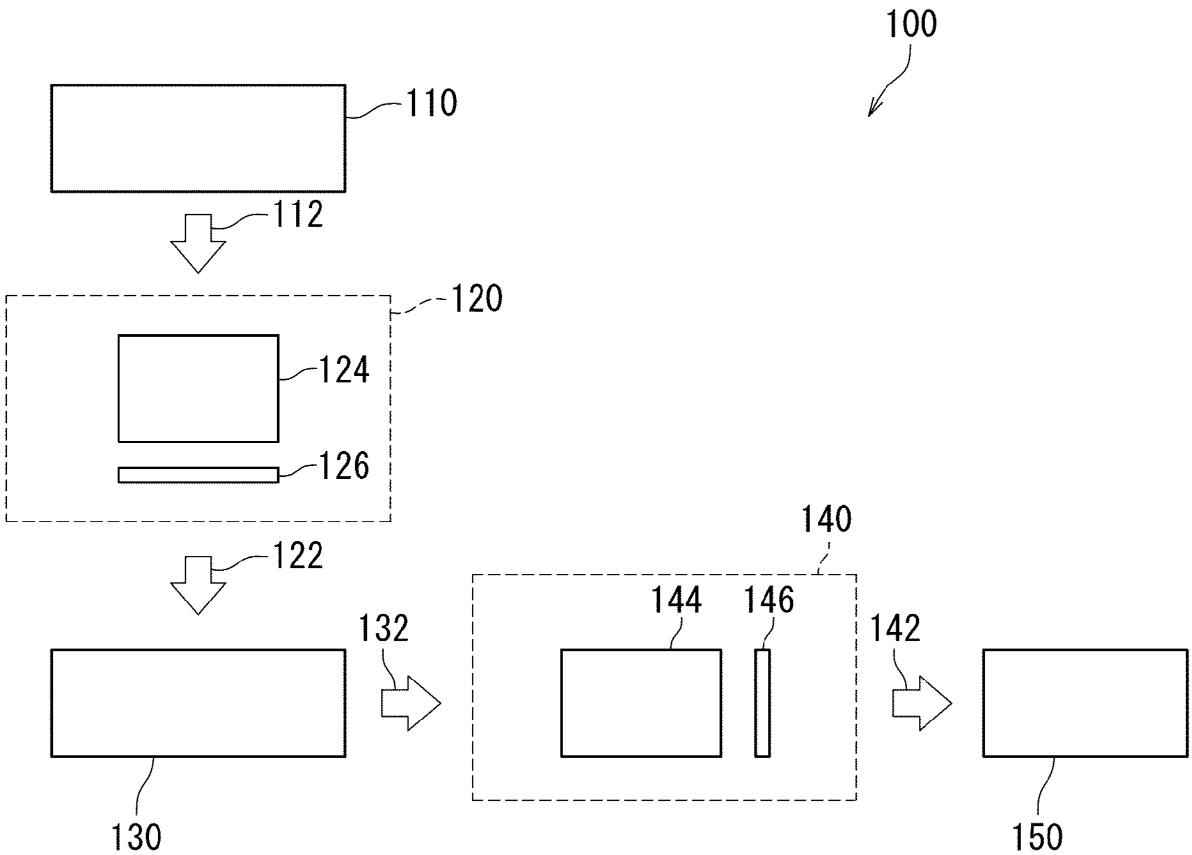

*Fig.14*

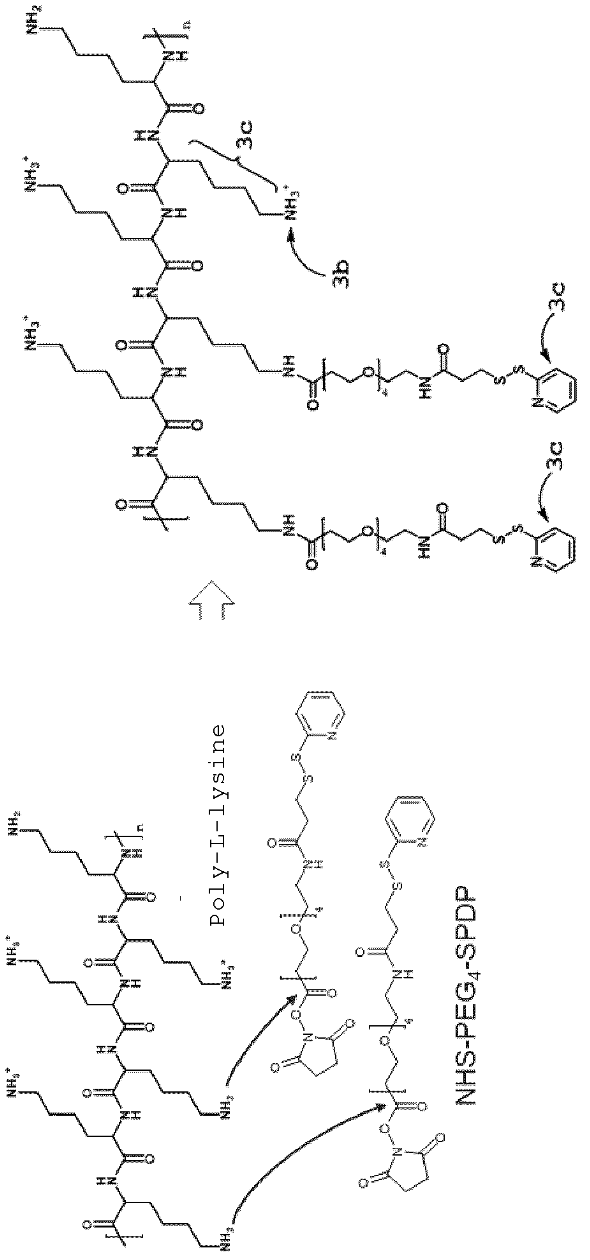

*Fig.15*

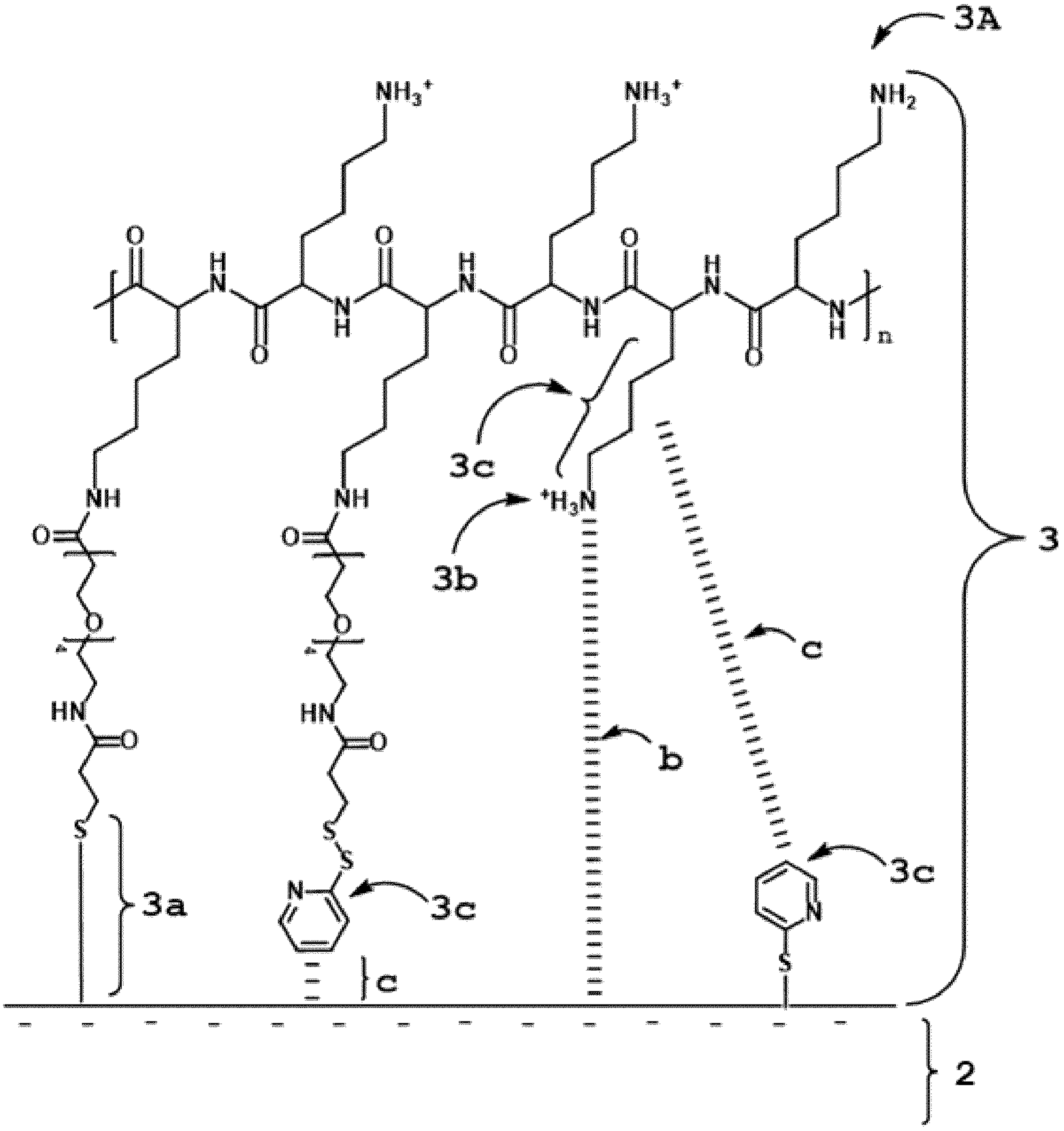

*Fig.16*

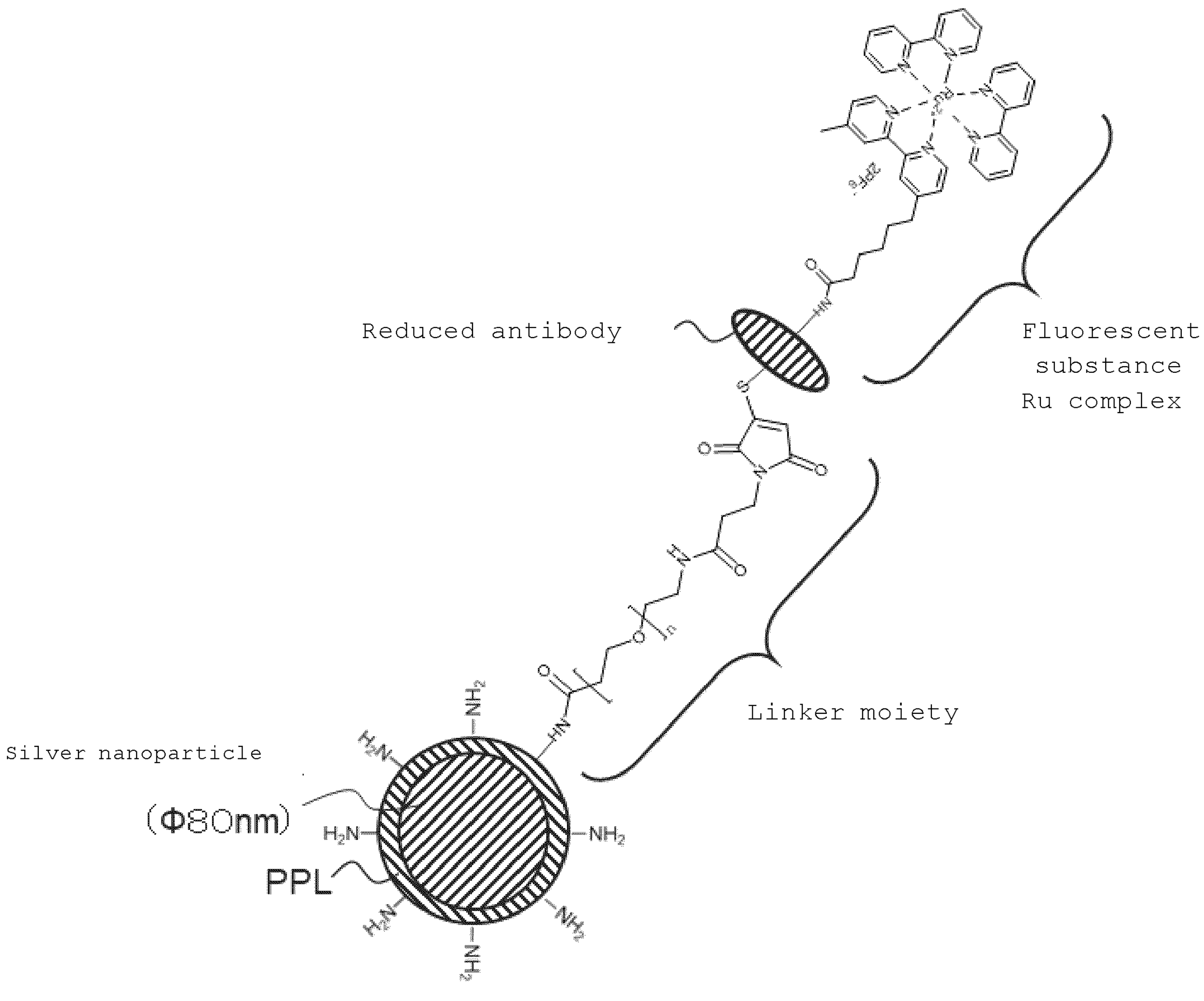
Reduced antibody
Fluorescent substance
Ru complex
Linker moiety
Silver nanoparticle
(Φ80nm)
PPL

Fig.17

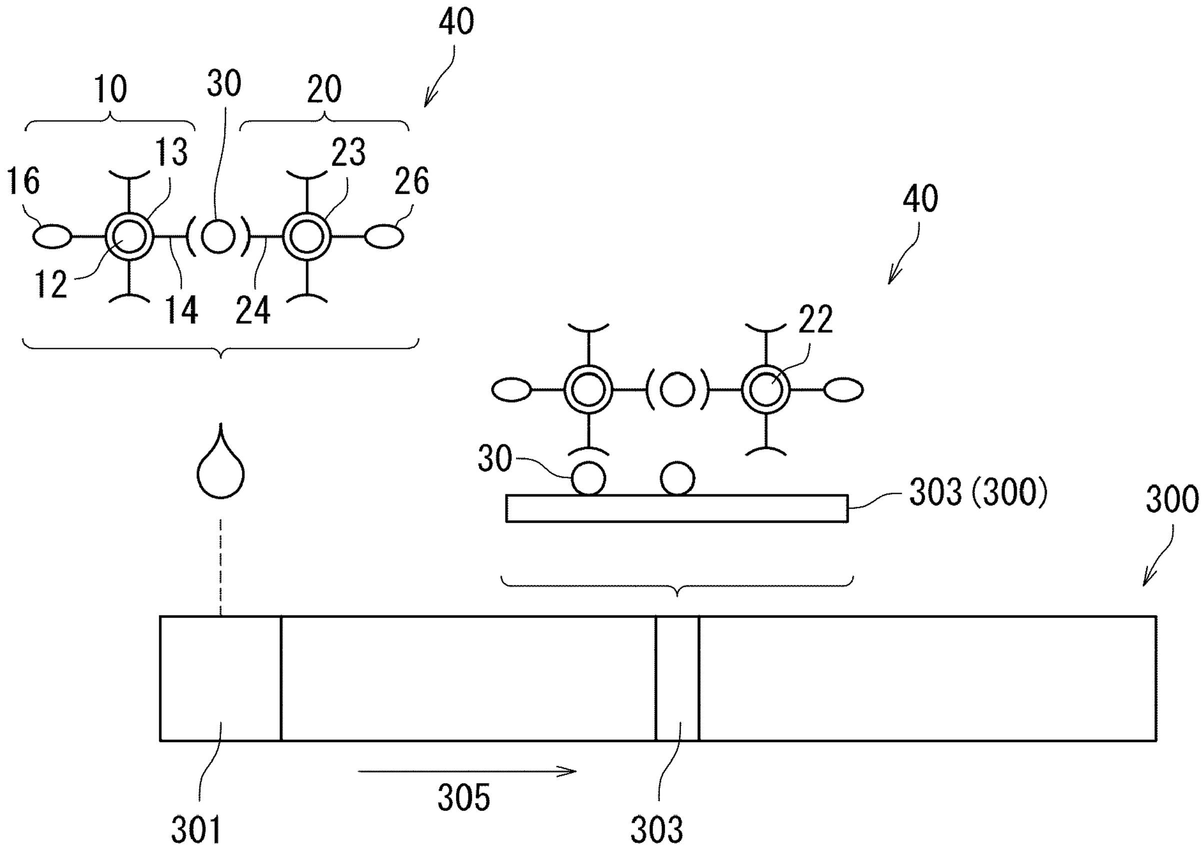

*Fig.18*

(a)

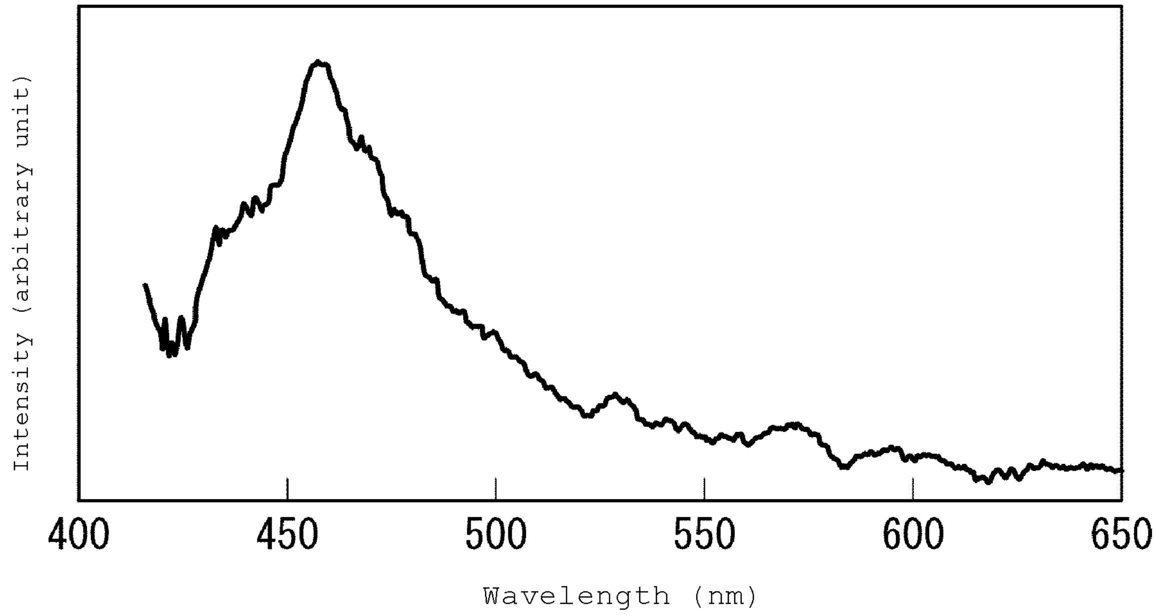
Intensity (arbitrary unit)
400
450
500
550
600
650
Wavelength (nm)

(b)

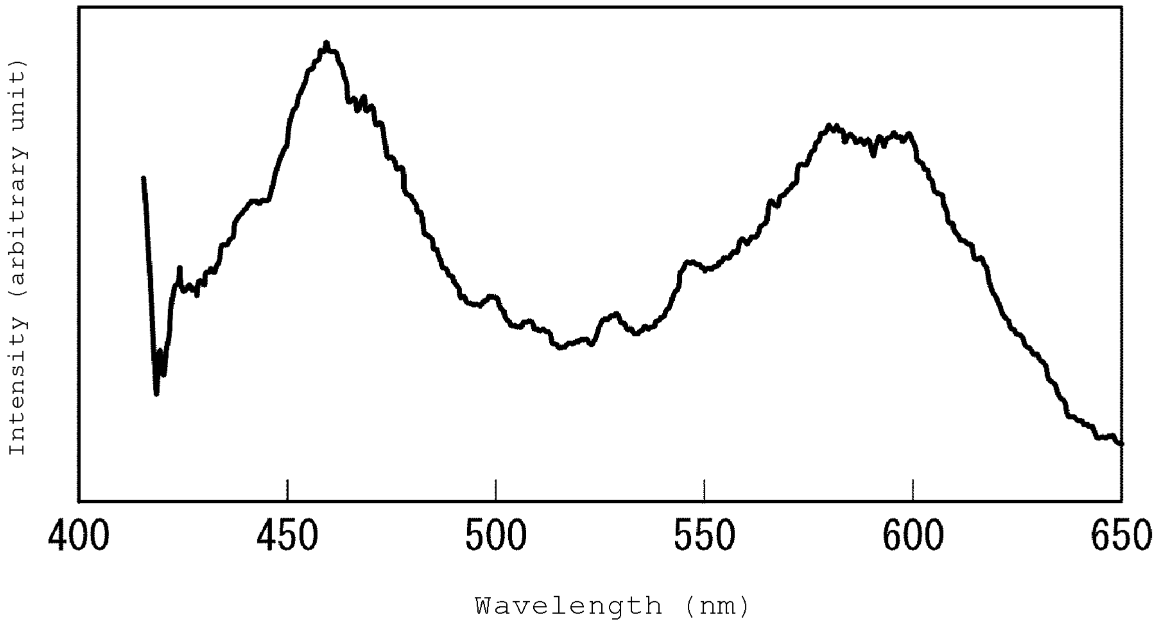
Intensity (arbitrary unit)
400
450
500
550
600
650
Wavelength (nm)

*Fig.19*

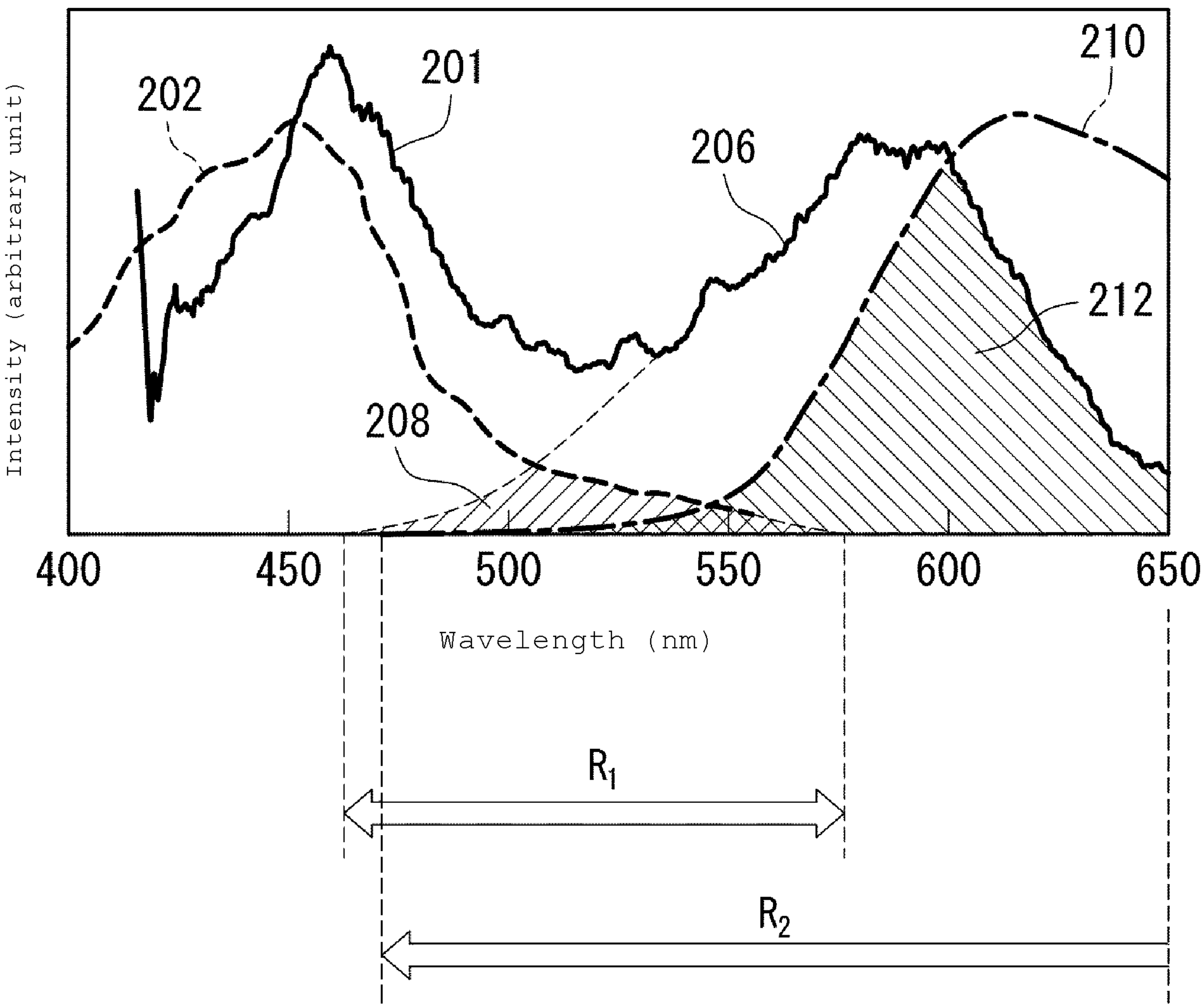
Intensity (arbitrary unit)
202
201
206
210
212
208
400
450
500
550
600
650
Wavelength (nm)
R1
R2

*Fig.20*

(a)

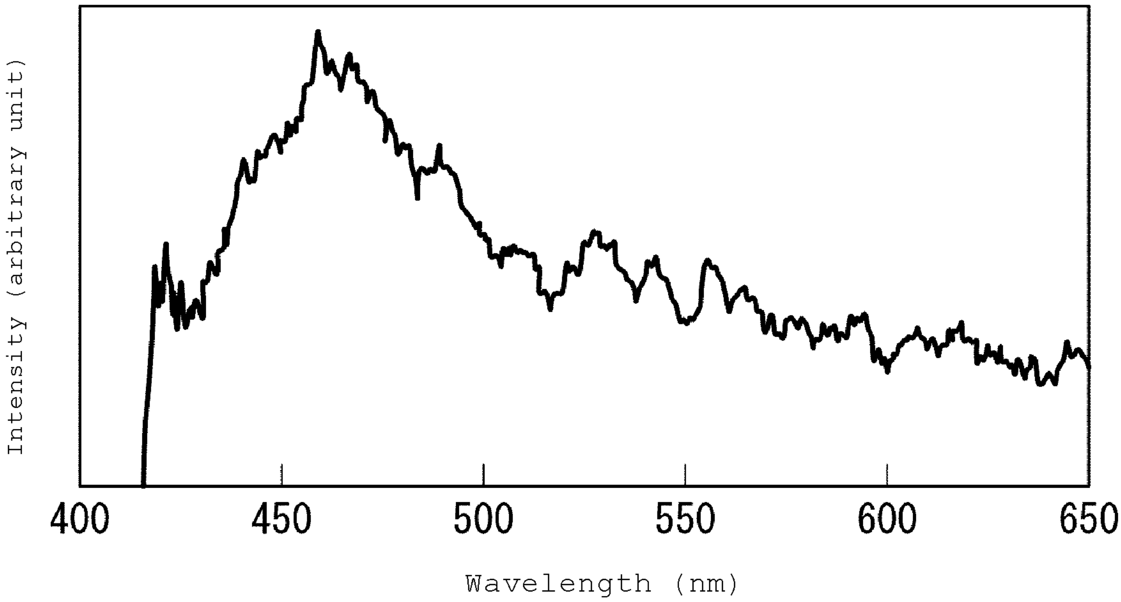
Intensity (arbitrary unit)
400
450
500
550
600
650
Wavelength (nm)

(b)

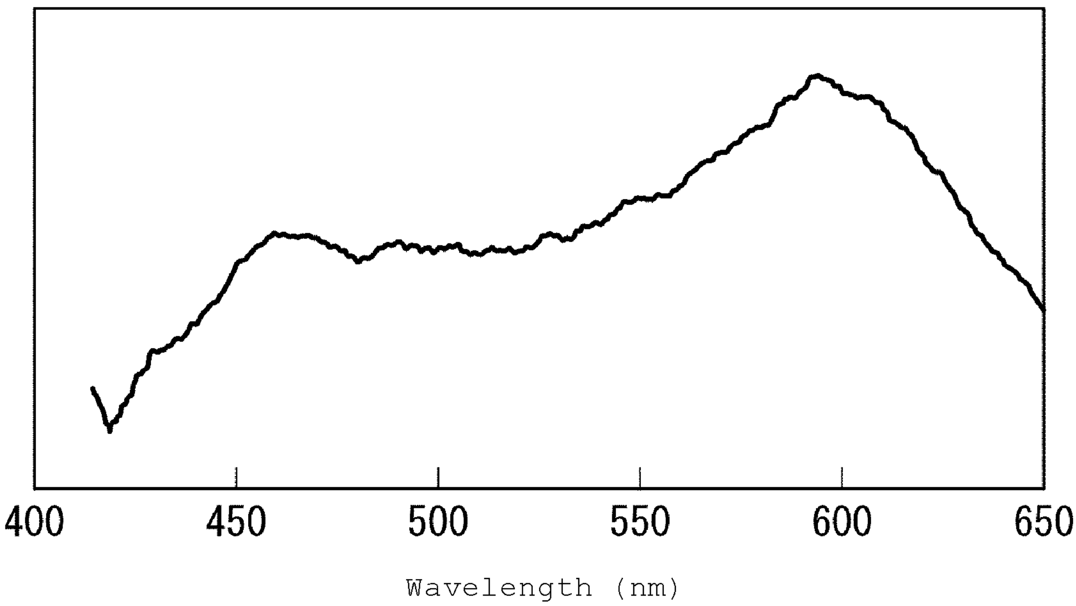
400
450
500
550
600
650
Wavelength (nm)

*Fig.21*

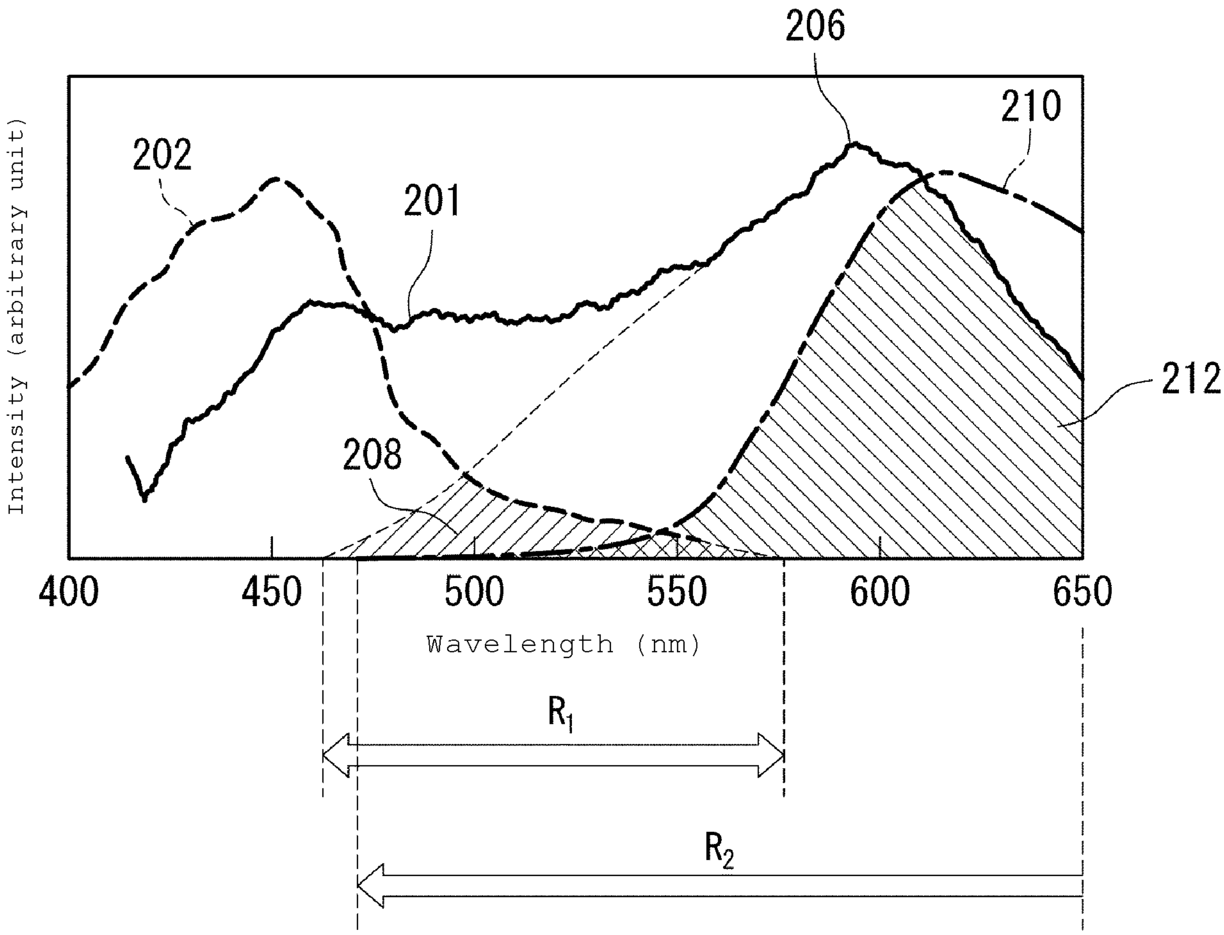

206
210
202
201
212
208
Intensity (arbitrary unit)
400
450
500
550
600
650
Wavelength (nm)
R1
R2

*Fig.22*

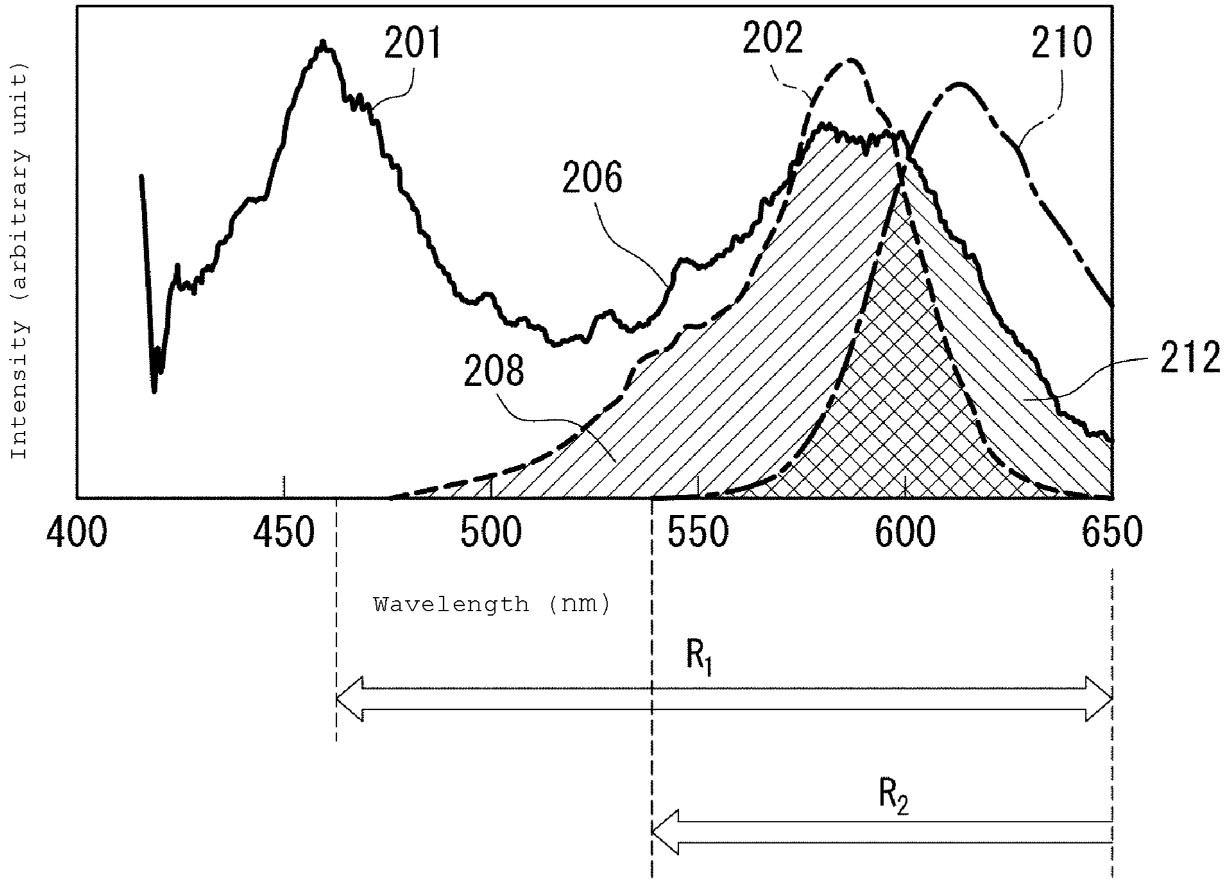
201
202
210
206
208
212
Intensity (arbitrary unit)
400
450
500
550
600
650
Wavelength (nm)
$R_1$
$R_2$

*Fig.23*

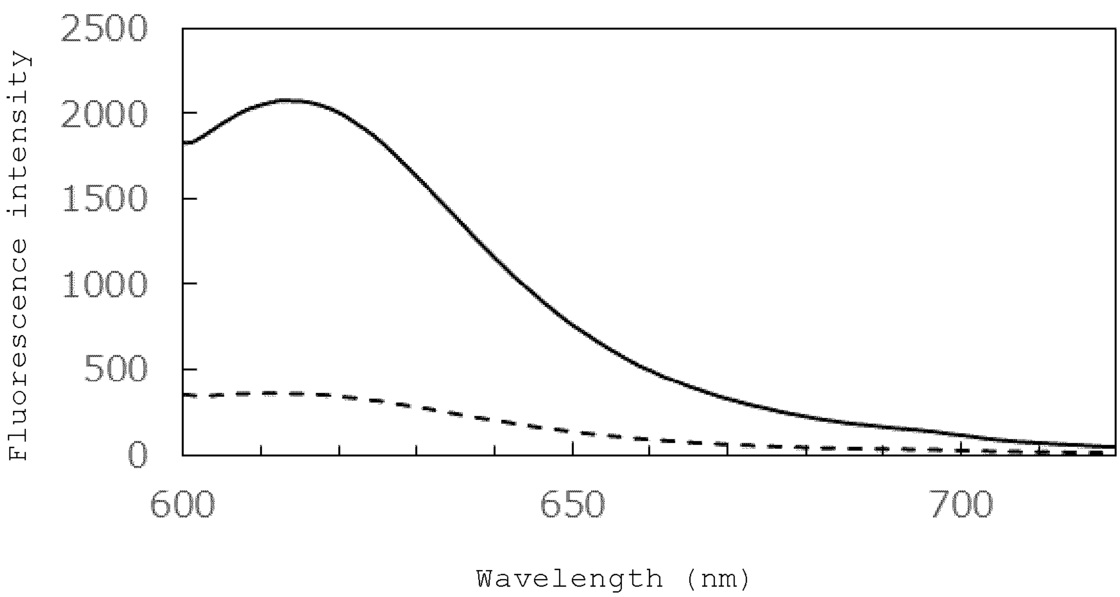
Fluorescence intensity
2500
2000
1500
1000
500
0
600
650
700
Wavelength (nm)

Fig.24

(a)

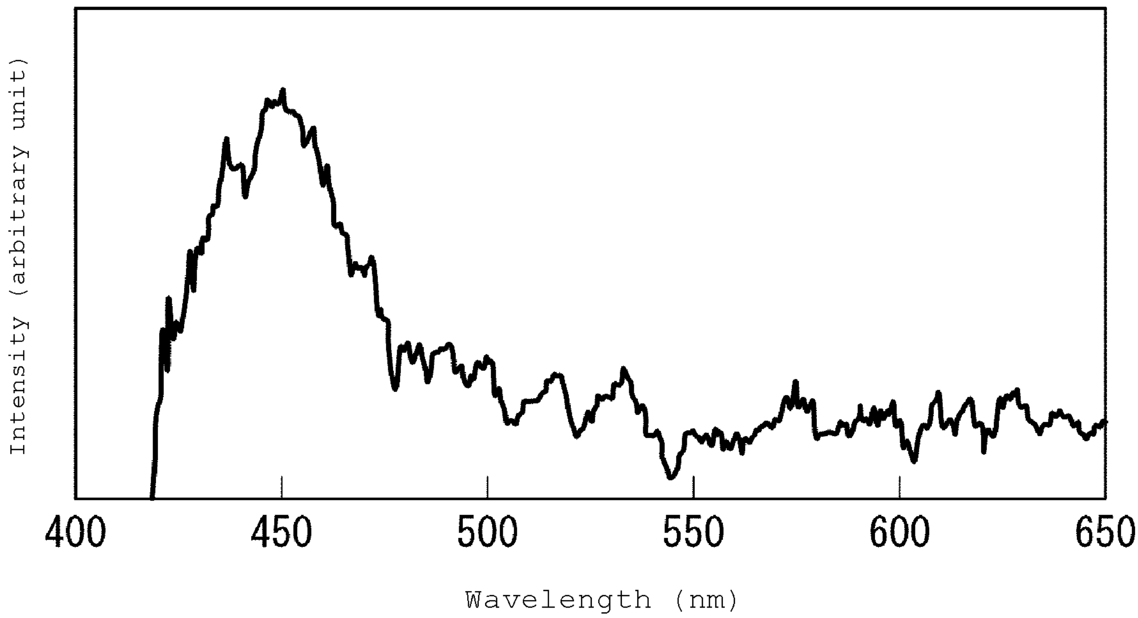
Intensity (arbitrary unit)
400
450
500
550
600
650
Wavelength (nm)

(b)

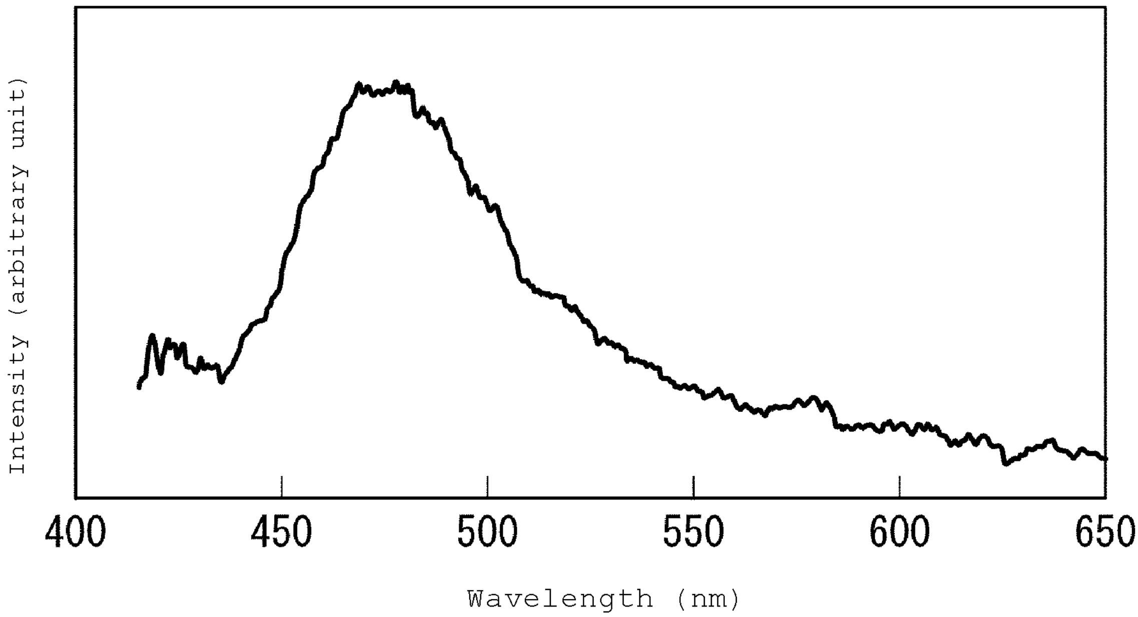
Intensity (arbitrary unit)
400
450
500
550
600
650
Wavelength (nm)

*Fig.25*

(a)

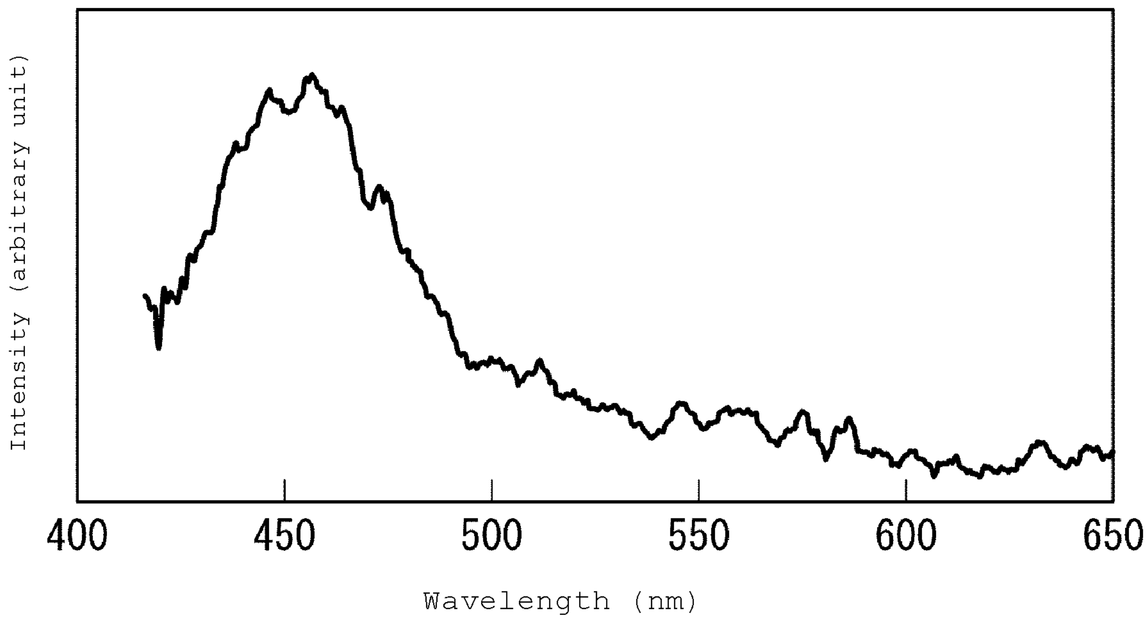
Intensity (arbitrary unit)
400
450
500
550
600
650
Wavelength (nm)

(b)

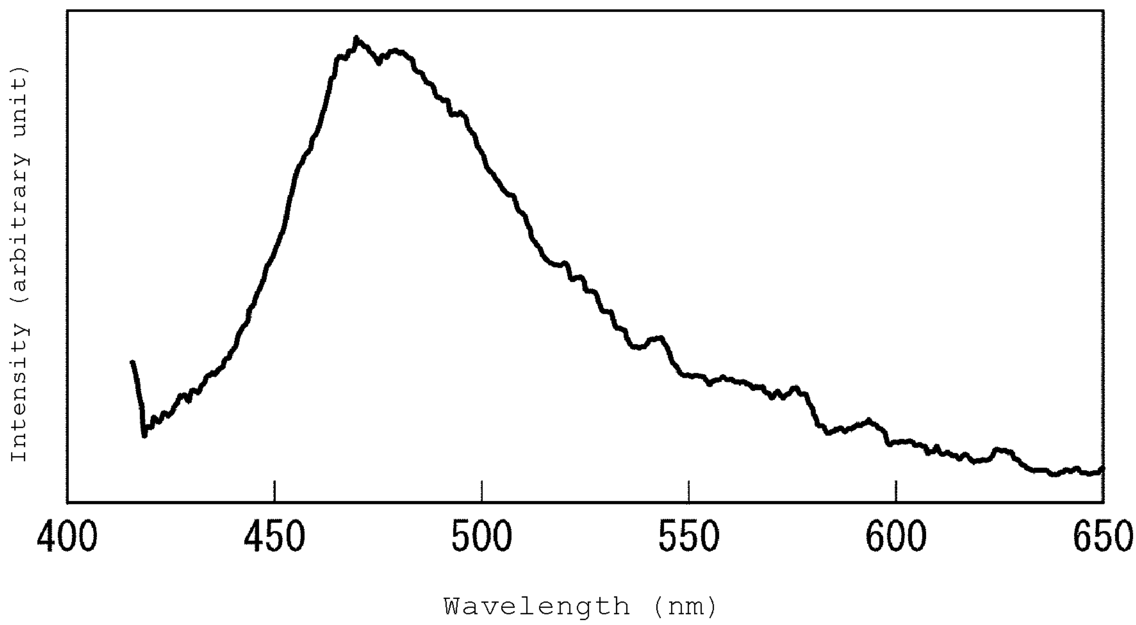
Intensity (arbitrary unit)
400
450
500
550
600
650
Wavelength (nm)

**INTERNATIONAL SEARCH REPORT**

International application No. **PCT/JP2023/021018**

**A. CLASSIFICATION OF SUBJECT MATTER**

***G01N 33/543***(2006.01)i; ***C09K 11/06***(2006.01)i; ***C09K 11/08***(2006.01)i; ***G01N 33/545***(2006.01)i; ***G01N 33/553***(2006.01)i
FI: G01N33/543 525C; G01N33/553; G01N33/545; G01N33/543 575; G01N33/543 595; C09K11/06 660; C09K11/08 G; C09K11/08 A

According to International Patent Classification (IPC) or to both national classification and IPC

**B. FIELDS SEARCHED**

Minimum documentation searched (classification system followed by classification symbols)

G01N33/543; C09K11/06; C09K11/08; G01N33/545; G01N33/553

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br><br>A | JP 2015-129773 A (KONICA MINOLTA, INC.) 16 July 2015 (2015-07-16)<br>paragraphs [0023], [0027]-[0029], [0093], [0094], fig. 2 | 25-26<br><br>1-24 |
| A | JP 2001-513198 A (THE REGENTS OF THE UNIVERSITY OF CALIFORNIA) 28 August 2001 (2001-08-28)<br>p. 25, lines 16-21 | 1-26 |
| A | JP 2022-512606 A (WASHINGTON UNIVERSITY) 07 February 2022 (2022-02-07)<br>paragraph [0218] | 1-26 |
| A | WO 2019/059171 A1 (PANASONIC INTELLECTUAL PROPERTY MANAGEMENT CO., LTD.) 28 March 2019 (2019-03-28)<br>claim 1 | 1-26 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 August 2023** | **29 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

**INTERNATIONAL SEARCH REPORT**
**Information on patent family members**

International application No.
**PCT/JP2023/021018**

| Patent document cited in search report | Publication date (day/month/year) | Patent family member(s) | Publication date (day/month/year) |
|---|---|---|---|
| JP 2015-129773 A | 16 July 2015 | WO 2011/096394 A1 | |
| JP 2001-513198 A | 28 August 2001 | US 6180415 B1<br>column 9, lines 57-59<br>US 2001/0002315 A1<br>US 2009/0161104 A1<br>US 2011/0244479 A1<br>US 2013/0038868 A1<br>US 2014/0209683 A1<br>WO 1998/037417 A1<br>EP 979409 A1 | |
| JP 2022-512606 A | 07 February 2022 | US 2021/0396747 A1<br>example 5<br>WO 2020/072924 A1<br>EP 3861343 A1<br>CN 113167791 A<br>CA 3115117 A1 | |
| WO 2019/059171 A1 | 28 March 2019 | US 2020/0166455 A1<br>claim 1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011220705 A